# EUROPEAN PATENT APPLICATION

(11) **EP 4 082 427 A1**
(43) Date of publication of application: **02.11.2022**
(21) Application number: 20897671.2
(22) Date of filing: 01.12.2020
(51) Int. Cl.: A61B 5/02, A61B 5/145

(54) **ELECTRONIC DEVICE**

(30) Priority: 25.12.2019 JP 2019234792
(71) Applicant: Kyocera Corporation, Kyoto-shi Kyoto 612-8501 (JP)
(72) Inventor: AJIMA Hiromi, Kyoto-shi Kyoto 6128501 (JP)
(74) Representative: Viering, Jentschura & Partner mbB Patent- und Rechtsanwälte
(86) International application number: PCT/JP2020/044696
(87) International publication number: WO 2021/131536

(57) **Abstract**

An electronic device includes a sensor capable of detecting pulsation in a target region of a subject; a housing including, at least in part, the sensor; a support portion that supports the housing on a side thereof; an elastic member interposed between the housing and the support portion; and a base portion that allows the support portion to stand upright.

## Description

### Cross Reference to Related Applications

The present application claims priority to Japanese Patent Application No. 2019-234792 filed in Japan on December 25, 2019, the entire disclosure of which is incorporated herein by reference.

### Technical Field

The present disclosure relates to an electronic device.

### Background Art

In the related art, there is known an electronic device that measures biological information from a target region of a subject, such as a wrist. For example, PTL 1 describes an electronic device wearable on a wrist of a subject to measure the pulse of the subject.

### Citation List

### Patent Literature

PTL 1: Japanese Unexamined Patent Application Publication No. 2002-360530

### Summary of Invention

An electronic device according to an embodiment includes
a sensor capable of detecting pulsation in a target region of a subject,
a housing including, at least in part, the sensor,
a support portion that supports the housing on a side thereof,
an elastic member interposed between the housing and the support portion, and
a base portion that allows the support portion to stand upright.

### Brief Description of Drawings

[Fig. 1] Fig. 1 is a diagram illustrating a manner of using an electronic device according to an embodiment.
[Fig. 2] Fig. 2 is a diagram describing a target region of a subject.
[Fig. 3] Fig. 3 is a diagram illustrating the appearance of an electronic device according to an embodiment.
[Fig. 4] Fig. 4 is a diagram illustrating the appearance of an electronic device according to an embodiment.
[Fig. 5] Fig. 5 is a diagram illustrating the appearance of an electronic device according to an embodiment.
[Fig. 6] Fig. 6 is a diagram illustrating the appearance of an electronic device according to an embodiment.
[Fig. 7] Fig. 7 is a diagram illustrating an electronic device according to an embodiment and a wrist of the subject.
[Fig. 8] Fig. 8 is a diagram illustrating a cross section of an electronic device according to an embodiment.
[Fig. 9] Fig. 9 is a diagram illustrating the cross section of an electronic device according to an embodiment.
[Fig. 10] Fig. 10 is a diagram illustrating a manner of using an electronic device according to an embodiment.
[Fig. 11] Fig. 11 is a diagram illustrating the appearance of an electronic device according to an embodiment.
[Fig. 12] Fig. 12 is a diagram illustrating the appearance of a wrist rest portion of a base portion of an electronic device according to an embodiment.
[Fig. 13] Fig. 13 is a diagram illustrating a cross section of the wrist rest portion of the base portion of an electronic device according to an embodiment.
[Fig. 14] Fig. 14 is a diagram illustrating the appearance of an electronic device according to an embodiment.
[Fig. 15] Fig. 15 is a diagram illustrating the appearance of an electronic device according to an embodiment.
[Fig. 16] Fig. 16 is a diagram illustrating the appearance of an electronic device according to an embodiment.
[Fig. 17] Fig. 17 is a diagram illustrating an electronic device according to an embodiment and the wrist of the subject.
[Fig. 18] Fig. 18 is a functional block diagram illustrating a schematic configuration of an electronic device according to an embodiment.
[Fig. 19] Fig. 19 is a diagram illustrating an example of a pulse wave acquired with a sensor unit.
[Fig. 20] Fig. 20 is a diagram illustrating a time variation in calculated AI.
[Fig. 21] Fig. 21 is a diagram illustrating a calculated AI and a measurement result of blood glucose level.
[Fig. 22] Fig. 22 is a diagram illustrating the relationship between the calculated AI and the blood glucose level.
[Fig. 23] Fig. 23 is a diagram illustrating a calculated AI and a measurement result of triglyceride value.
[Fig. 24] Fig. 24 is a flowchart illustrating a procedure for estimating blood fluidity and the states of glucose metabolism and lipid metabolism.
[Fig. 25] Fig. 25 is a schematic diagram illustrating a schematic configuration of a system according to an embodiment.

### Description of Embodiments

An electronic device capable of easily measuring biological information of a subject can improve its usability. It is an object of the present disclosure to provide an electronic device with high usability. According to the present disclosure, it is possible to provide an electronic device with improved usability. An embodiment will be described hereinafter in detail with reference to the drawings.

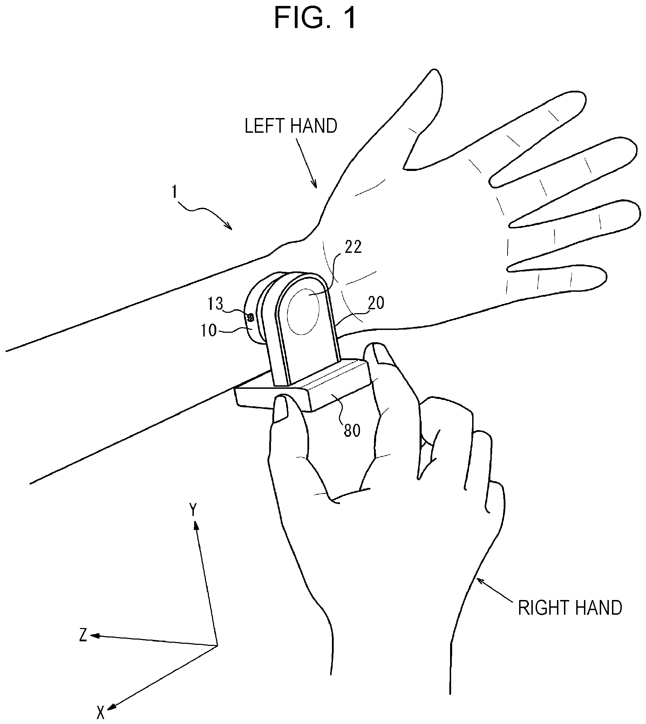

Fig. 1 is a diagram describing a manner of using an electronic device according to an embodiment. That is, Fig. 1 is a diagram illustrating how a subject measures biological information by using an electronic device according to an embodiment.

As illustrated in Fig. 1, an electronic device 1 according to an embodiment is capable of measuring biological information of a subject from, for example, a portion of the subject, such as a wrist of the subject, as a target region. In the example illustrated in Fig. 1, the electronic device 1 remains in abutment against a target region of the left wrist of the subject. In the example illustrated in Fig. 1, the electronic device 1 remains in abutment against a target region, the target region being a wrist portion present on the way from the palm toward the elbow of the left hand of the subject. As described below, for example, in a state where the electronic device 1 does not come into abutment against the target region of the subject, such as before measurement, the electronic device 1 can be erected on a horizontal surface such as on a table or a desk in a free-standing manner.

As illustrated in Fig. 1, the electronic device 1 according to an embodiment includes a housing 10, a support portion 20, and a base portion 80. The housing 10 may include a switch 13 that turns on/off the power supply of the electronic device 1. As described below, the housing 10 includes a sensor 50 capable of detecting pulsation in the target region of the subject. The support portion 20 includes a rear surface portion 22, and this portion may be pressed by the subject or the like. As described below, the support portion 20 may include an extension portion 24 that is extendable. The base portion 80 supports the support portion 20 in an upright state. The functional units of the electronic device 1 will further be described below.

The positive direction of the Y axis illustrated in Fig. 1 is also referred to as an "upward" direction, if necessary. The negative direction of the Y axis illustrated in Fig. 1 is also referred to as a "downward" direction, if necessary. That is, the upward direction and the downward direction illustrated in Fig. 1 may be substantially the same as the upward direction and the downward direction when viewed from the viewpoint of the subject, respectively.

In Fig. 1, a portion of the electronic device 1 viewed from a viewpoint directed to the positive direction of the Z axis is referred to as a "rear surface" of the electronic device 1. That is, in Fig. 1, the rear surface of the electronic device 1 is a portion of the support portion 20 of the electronic device 1 where the rear surface portion 22 is viewed in plan. In Fig. 1, a portion of the electronic device 1 viewed from a viewpoint directed to the negative direction of the Z axis is referred to as a "front surface" of the electronic device 1. That is, in Fig. 1, that is, in Fig. 1, the front surface of the electronic device 1 is a portion of the housing 10 of the electronic device 1 where a surface to be brought into abutment against the target region of the subject is viewed in plan.

Before the measurement of biological information of the subject using the electronic device 1 illustrated in Fig. 1, the following preparation may be carried out, for example. First, the subject may place the arm to be subjected to measurement of the biological information (in the example illustrated in Fig. 1, the left arm of the subject) on, for example, a stable base or the like such as a table or a desk. In Fig. 1, the base described above, such as a table or a desk, may have a deck (top plate) parallel to the XZ plane (i.e., perpendicular to the Y axis) illustrated in the drawing, for example. That is, the subject may place the arm to be subjected to measurement of the biological information on a base or the like having a top plate perpendicular to the Y axis illustrated in the drawing. At this time, the palm of the hand to be subjected to measurement of the biological information of the subject (the left hand illustrated in Fig. 1) may be directed toward the negative direction of the Z axis illustrated in the drawing or directed toward a direction slightly shifted to the positive direction of the Y axis from the negative direction of the Z axis.

Then, the subject may place the electronic device 1 on, for example, a stable base or the like such as a table or a desk so that the electronic device 1 can be erected in a free-standing manner. To erect the electronic device 1 in a free-standing manner, for example, the subject may place the electronic device 1 such that the bottom surface portion of the base portion 80 is placed on the deck (top plate) of the base described above, such as a table or a desk. At this time, the subject may bring the housing 10 of the electronic device 1 into abutment against the target region so that the sensor 50 of the electronic device 1 is located at a position where pulsation in the target region can be satisfactorily detected. Alternatively, the subject may bring the target region into abutment against the housing 10 of the electronic device 1. In this case, the subject may position the electronic device 1 using the hand not to be subjected to measurement of the biological information (in the example illustrated in Fig. 1, the right hand of the subject).

Then, as illustrated in Fig. 1, the subject may press the base portion 80 of the electronic device 1 against the deck (top plate) of the base, such as a table or a desk, using a finger or the like of the hand not to be subjected to measurement of the biological information (in the example illustrated in Fig. 1, the right hand of the subject). As a result, the position of the electronic device 1 is secured on the table or desk. In the example illustrated in Fig. 1, the base portion 80 of the electronic device 1 is pressed against the table or desk by the subject with the thumb and index finger of the right hand. The base portion 80 stands upright, with the support portion 20 secured. Accordingly, as illustrated in Fig. 1, the electronic device 1 according to an embodiment measures biological information of the subject while being pressed against the target region. The fingers with which the subject presses the base portion 80 against the table or desk are not limited to the thumb and index finger of the right hand. The subject may press the base portion 80 against the table or desk with a finger other than the thumb and index finger of the right hand. Further, the subject may not necessarily press the base portion 80 of the electronic device 1 against the table or desk, but may press, for example, the support portion 20 or the like against the table or desk. The base portion 80 or the support portion 20 of the electronic device 1 may be pressed in any manner if it is pressed against the table or desk with an appropriate pressing force. The base portion 80 or the support portion 20 of the electronic device 1 may be placed on a table, a desk, or any other base made of wood, iron, plastic, glass, rubber, resin, or any other material, and any combination thereof.

The electronic device 1 can detect pulsation in the target region upon being brought into abutment against the target region of the subject. The target region of the subject may be, for example, a region of the body where the ulnar artery or radial artery of the subject is present beneath the skin. The target region of the subject is not limited to a region of the body where the ulnar artery or radial artery of the subject is present beneath the skin, and may be any region of the body where the pulsation of the subject is detectable. Fig. 1 illustrates a state in which the electronic device 1 remains in abutment against a target region, the target region being a region of the body where the radial artery is located beneath the skin of the wrist of the subject.

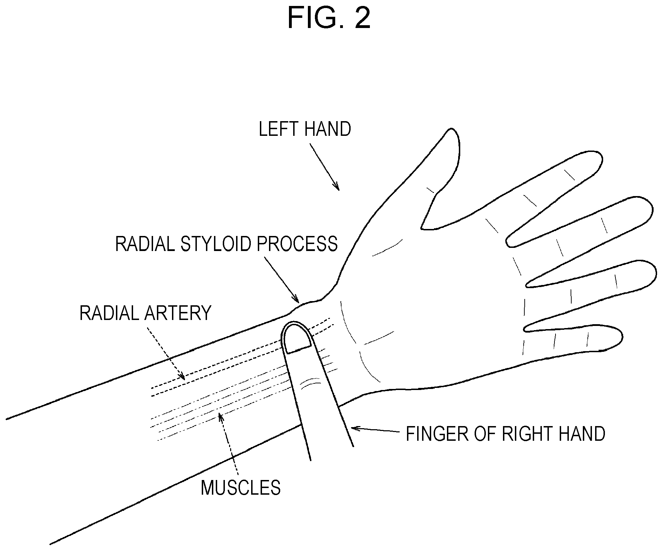

Fig. 2 is a diagram describing the target region of the subject. More specifically, Fig. 2 illustrates an example in which the subject searches their target region for a portion where pulsation is satisfactorily detectable before measuring biological information using the electronic device 1. That is, Fig. 2 illustrates how the subject searches the target region of their left hand for a portion where pulsation is satisfactorily detectable, using a finger of their right hand. In Fig. 2, as in Fig. 1, it may be assumed that the subject has placed their left arm on a base or the like such as a table or a desk. In Fig. 2, the radial artery and the muscles beneath the skin of the arm of the subject are indicated by broken lines or chain lines, and the like.

As described above, the subject may bring the housing 10 of the electronic device 1 into abutment against the target region such that the sensor 50 of the electronic device 1 is located at a position where pulsation is satisfactorily detectable. The position on the target region of the subject where pulsation is satisfactorily detectable differs depending on the subject (individual difference). Accordingly, the subject may search their target region for a position where pulsation is satisfactorily detectable before measuring biological information using the electronic device 1.

In many cases, the position where pulsation is satisfactorily detectable near the wrist of the subject is a position where the radial artery runs beneath the skin and where the radial styloid process is present beneath the skin, or near this position. In a portion where the radial artery runs above the radial styloid process, the radial artery is located above the radial styloid process, which is relatively stiff. At this position, the movement of the radial artery that contracts due to pulsation is more easily transmitted toward the skin of the subject, which is relatively soft, than toward the radial styloid process, which is relatively stiff. Accordingly, the position described above may be set as the target region for the measurement of biological information of the subject using the electronic device 1 according to an embodiment.

As illustrated in Fig. 2, it is assumed that the subject has found a good pulse at, for example, a position illustrated in the drawing around the wrist of their left hand using a fingertip of their right hand. In this case, the subject may set the position at which the subject has found a good pulse using the fingertip of their right hand as the target region. In this way, as illustrated in Fig. 1, the subject may bring the housing 10 of the electronic device 1 into abutment against the target region. Setting the target region such that the target region includes the positions of many muscles illustrated in Fig. 2 may make it difficult to satisfactorily transmit the pulsation of the radial artery to the housing 10 (and the sensor 50) of the electronic device 1. Accordingly, when bringing the housing 10 of the electronic device 1 into abutment against the target region, the subject may arrange the electronic device 1 so that the housing 10 (and the sensor 50) of the electronic device 1 can be pressed against the radial artery while avoiding the muscles as much as possible. A portion of the housing 10 of the electronic device 1 to be brought into abutment against the target region of the subject will further be described below. Further, as illustrated in Fig. 1, when measuring biological information of the subject using the electronic device 1, the subject may be in a psychological condition such that the entire body is relaxed, and the palm of the hand to be subjected to measurement of the biological information (for example, the left hand) may be slightly opened.

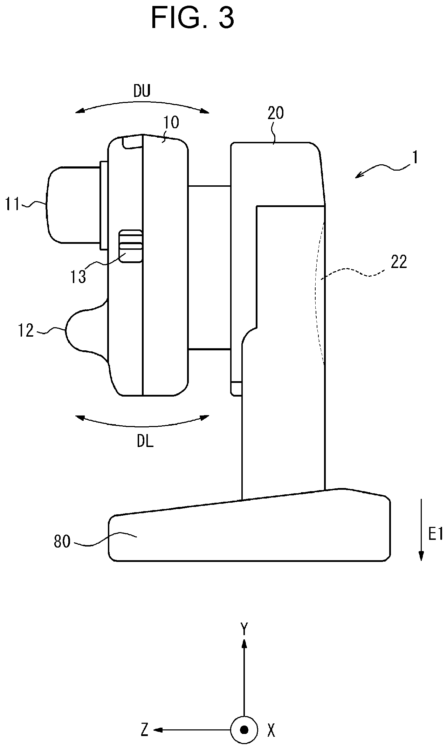

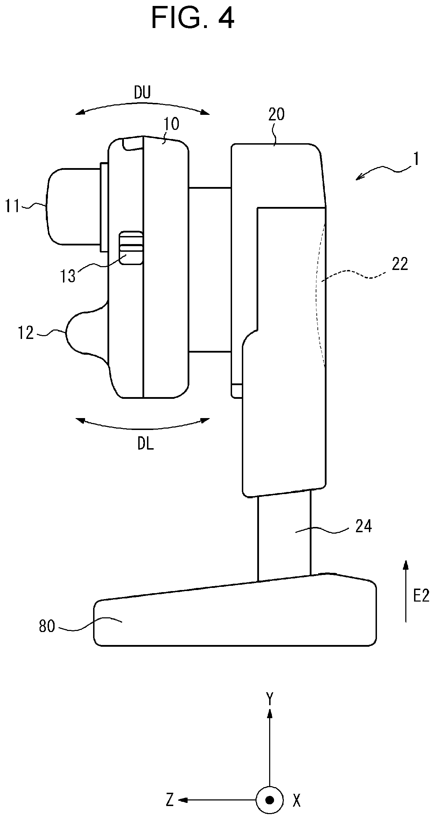

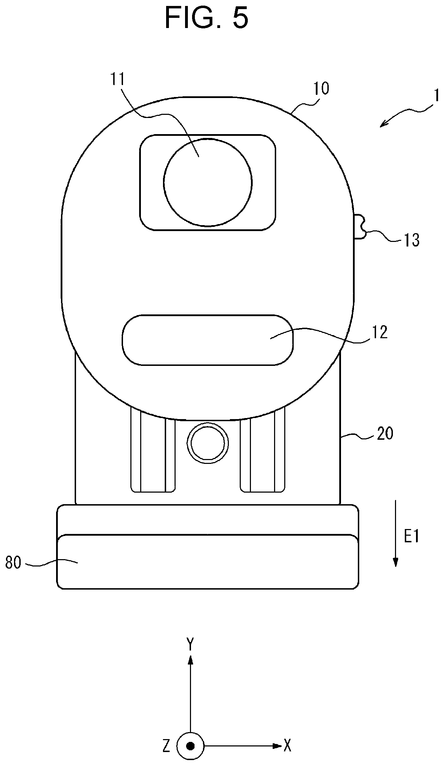

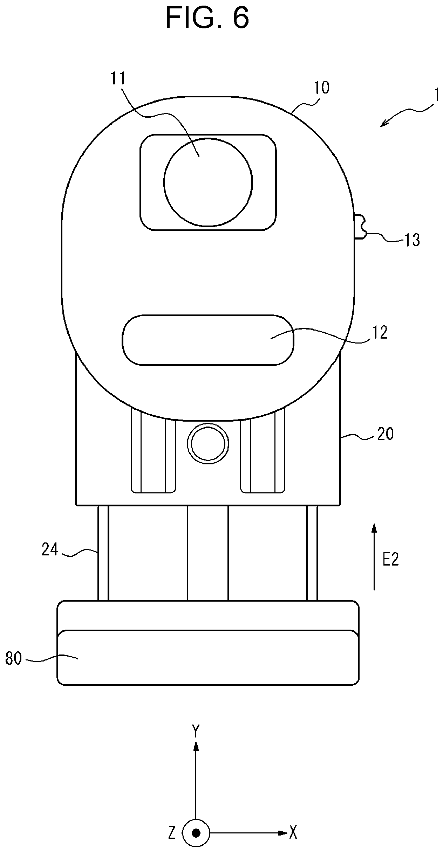

Next, the configuration of the electronic device 1 according to an embodiment will further be described. Fig. 3 and Fig. 4 are diagrams illustrating the electronic device 1 illustrated in Fig. 1 when viewed from a viewpoint directed to the negative direction of the X axis. That is, Fig. 3 and Fig. 4 are diagrams illustrating the right side surface of the electronic device 1 illustrated in Fig. 1. Fig. 5 and Fig. 6 are diagrams illustrating the electronic device 1 illustrated in Fig. 1 when viewed from a viewpoint directed to the negative direction of the Z axis. That is, Fig. 5 and Fig. 6 are diagrams illustrating the front surface of the electronic device 1 illustrated in Fig. 1.

As illustrated in Fig. 3 to Fig. 6, the electronic device 1 is configured to include the housing 10, the support portion 20, and the base portion 80. In the electronic device 1, as described below, the housing 10 and the support portion 20 are connected through an elastic member. As illustrated in Fig. 3 to Fig. 6, the support portion 20 supports the housing 10 on a side of the support portion 20. That is, the housing 10 is supported on a side of the support portion 20. The base portion 80 allows the support portion 20 to stand upright. The housing 10, the support portion 20, and/or the base portion 80 may be made of, for example, a material such as ceramic, iron, any other metal, resin, plastic, or aluminum. The housing 10, the support portion 20, and/or the base portion 80 may be made of a hard and lightweight material. The material of the housing 10, the support portion 20, and/or the base portion 80 is not limited, and may have strength enough to function as a measurement device. Further, the material of the housing 10, the support portion 20, and/or the base portion 80 is not excessively heavy and may be relatively light.

The sizes of the housing 10, the support portion 20, and the base portion 80 of the electronic device 1 are not limited, and may be relatively small in terms of portability and/or ease of measurement or the like. For example, the electronic device 1 may have a size such that, for example, the entire electronic device 1 is included in a cube or a rectangular parallelepiped having sides of about 7 cm each. However, in one embodiment, the size of the entire electronic device 1 may be larger or smaller than the size described above. Further, the shapes of the individual portions of the electronic device 1, such as the housing 10, the support portion 20, and the base portion 80, are not limited to the illustrated shapes, and various shapes may be used in terms of functionality of a measurement device and/or design viewpoint or the like. In particular, the base portion 80 allows the support portion 20 to stand upright. Accordingly, the base portion 80 may be shaped to have a bottom area such that the electronic device 1 including the housing 10 and the support portion 20 can stand upright. Alternatively, the base portion 80 may have a bottom area such that the electronic device 1 can be erected on a horizontal surface in a free-standing manner.

As described below, the housing 10 and the support portion 20 can move freely to some extent with respect to each other. That is, in the electronic device 1, even in a state where the housing 10 is secured, the support portion 20 can move freely to some extent. In the electronic device 1, even in a state where the support portion 20 is secured, the housing 10 can move freely to some extent. For example, as illustrated in Fig. 3 and Fig. 4, in the electronic device 1, the housing 10 can move freely to some extent in a direction indicated by an arrow DU and/or a direction indicated by arrow DL illustrated in the drawing.

As illustrated in Fig. 3 to Fig. 6, the support portion 20 of the electronic device 1 may include, for example, the extension portion 24 therein. The extension portion 24 is configured to be extendable from the support portion 20. Fig. 3 and Fig. 5 illustrate a state in which the extension portion 24 is not extended from the support portion 20. In contrast, Fig. 4 and Fig. 6 illustrate a state in which the extension portion 24 is extended from the support portion 20. That is, in Fig. 3 and Fig. 5, the extension portion 24 is extended in a direction indicated by an arrow E1, thus making it possible to extend the extension portion 24 such that, as illustrated in Fig. 4 and Fig. 6, the extension portion 24 projects from the support portion 20. In contrast, in Fig. 4 and Fig. 6, the extension portion 24 is contracted in a direction indicated by an arrow E2, thus making it possible to return the extension portion 24 to the original position, as illustrated in Fig. 3 and Fig. 5. In the electronic device 1 according to an embodiment, therefore, the extension portion 24 may be extended or contracted to make the length of the support portion 20 in the upward/downward direction adjustable.

In addition, the length of the support portion 20 in the upward/downward direction can be adjusted by the extension portion 24 to make the position of the housing 10 in the upward/downward direction (height direction) adjustable. Accordingly, even if the thickness of the left wrist of the subject illustrated in Fig. 1 differs to some extent from individual to individual, the position at which the housing 10 is brought into abutment against the target region of the subject can be adjusted in accordance with the position of the target region of the subject in the upward/downward direction. In this manner, in the electronic device 1 according to an embodiment, the support portion 20 may be configured to be extendable or contractible in a predetermined direction, such as the direction indicated by the arrow E1 and/or the arrow E2, to make the position of the housing 10 in the height direction adjustable.

The extension portion 24 may be configured to be extendable steplessly from the support portion 20. That is, the extension portion 24 may be configured such that the extension portion 24 can be positioned at any position up to a predetermined length, for example. With this configuration, even if the thickness of the wrist of the subject, including the target region, differs from individual to individual, the position at which the housing 10 of the electronic device 1 is brought into abutment against the target region of the subject can be finely adjusted.

The extension portion 24 may be configured to be extendable stepwise from the support portion 20. That is, the extension portion 24 may be configured to include, for example, a mechanism that facilitates positioning at a plurality of predetermined positions up to a predetermined length. For example, the extension portion 24 may include a mechanism such as a multi-stage stay that is locked in multiple stages when the extension portion 24 is extended or contracted from the support portion 20. With this configuration, when the subject measures biological information using the electronic device 1, for example, the same measurement environment as that of the previous measurement is easily reproduced. In this manner, in the electronic device 1 according to an embodiment, for example, the support portion 20 may be provided with the extension portion 24 to thereby be configured to be extendable or contractible stepwise in a predetermined direction, such as the direction indicated by the arrow E1 and/or the arrow E2.

As illustrated in Fig. 3 to Fig. 6, the housing 10 of the electronic device 1 may include a first abutment portion 11 as a portion to be brought into abutment against the target region of the subject. The first abutment portion 11 may be disposed on the side of the housing 10 closer to the target region. The first abutment portion 11 may function as a member such as a pulse contact portion, for example. As illustrated in Fig. 3 to Fig. 6, the housing 10 of the electronic device 1 may further include a second abutment portion 12 as a portion to be brought into abutment against the target region of the subject or the vicinity of the target region. The second abutment portion 12 may be brought into abutment against the vicinity of the position against which the first abutment portion 11 abuts in the target region of the subject. The second abutment portion 12 may also be disposed on the side of the housing 10 closer to the target region (side closer to the wrist of the subject).

As described above, the first abutment portion 11 is a member to be appropriately brought into abutment against the target region of the subject when the electronic device 1 measures biological information of the subject. Accordingly, the first abutment portion 11 may have a size such that, for example, the first abutment portion 11 is appropriately brought into abutment against a region of the body where the ulnar artery or radial artery of the subject is present beneath the skin. For example, as illustrated in Fig. 5 and Fig. 6, the first abutment portion 11 may have a width of about 1 cm to 1.5 cm in the X-axis direction or the Y-axis direction. Alternatively, the first abutment portion 11 may have a width other than about 1 cm to 1.5 cm in the X-axis direction or the Y-axis direction.

The first abutment portion 11 and the second abutment portion 12 may be made of, for example, a material such as ceramic, iron, any other metal, resin, plastic, or aluminum. The first abutment portion 11 and the second abutment portion 12 may be made of a hard and lightweight material. The material of the first abutment portion 11 and the second abutment portion 12 is not limited to any specific one. The material of the first abutment portion 11 and the second abutment portion 12 may have strength enough to function as a measurement device and may be relatively lightweight, like the housing 10 and/or the support portion 20.

As illustrated in Fig. 3 to Fig. 6, the housing 10 of the electronic device 1 may include the switch 13. The switch 13 may be, for example, a switch that switches on/off of the power supply of the electronic device 1. The switch 13 may be, for example, a switch that causes the electronic device 1 to start measurement of biological information. Fig. 3 to Fig. 6 illustrate an example in which the switch 13 is constituted by a slide switch. However, the switch 13 may be constituted by any switch such as a push button switch, for example. For example, when the switch 13 is constituted by a push button switch, various operations of the electronic device 1 may be supported in accordance with the number of times the switch 13 is pressed and/or the time during which the switch 13 is pressed, or the like. The location where the switch 13 is arranged is not limited to that in the example illustrated in Fig. 3 to Fig. 6, and the switch 13 may be arranged in any location. For example, the switch 13 may be arranged in the support portion 20.

Next, measurement of biological information using the electronic device 1 according to an embodiment will be described.

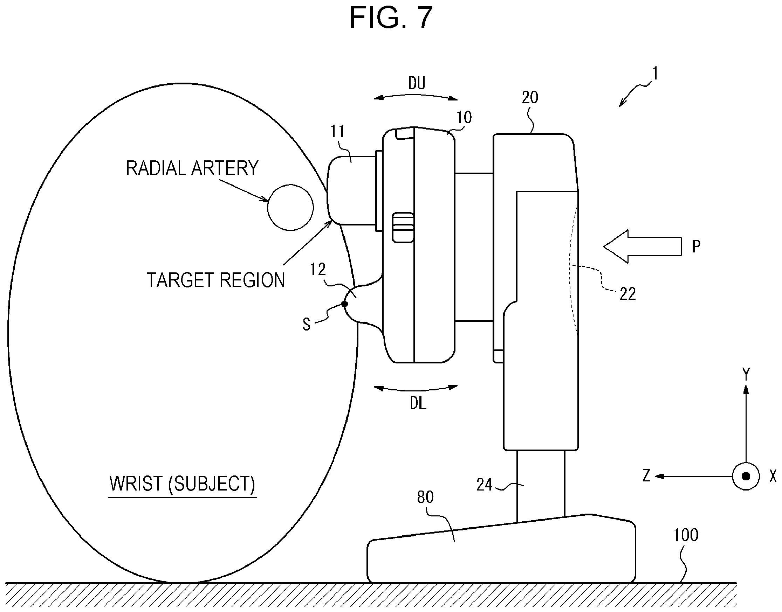

Fig. 7 illustrates how the subject measures biological information using the electronic device 1. Fig. 7 is a diagram illustrating the electronic device 1 illustrated in Fig. 1 when viewed from a side, together with a cross section of the wrist of the subject. That is, Fig. 7 is a diagram illustrating the electronic device 1 illustrated in Fig. 1 when viewed from a viewpoint directed to the negative direction of the X axis, together with a cross section of the wrist of the subject.

As illustrated in Fig. 7, the left wrist of the subject is placed on an upper surface of a deck (top plate) 100 of a base such as a table or a desk. The deck (top plate) 100 of the base, such as a table or a desk, is also referred to simply as a "horizontal surface 100". The horizontal surface 100 may be a surface that is horizontal, and may include not only a surface that is exactly horizontal but also a surface that is substantially horizontal. As illustrated in Fig. 7, furthermore, the electronic device 1 is erected on the horizontal surface 100 in a free-standing manner such that a lower end, that is, a bottom surface, of the base portion 80 that allows the support portion 20 to stand upright comes into contact with the horizontal surface 100. That is, in the electronic device 1 according to an embodiment, the base portion 80 may allow the support portion 20 to stand upright. The base portion 80 may be configured to allow the support portion 20 to stand upright such that the electronic device 1 is erected on the horizontal surface 100 in a free-standing manner. The example illustrated in Fig. 7 illustrates a state in which the extension portion 24 is somewhat extended from the support portion 20 of the electronic device 1. For example, the electronic device 1 can start measurement of biological information in a state where the base portion 80 (or the support portion 20) is pressed against the horizontal surface 100 by the subject with the right hand or the like. Alternatively, the electronic device 1 may be used without the bottom surface of the base portion 80 coming into contact with the upper surface of the horizontal surface 100 (i.e., in a state where the base portion 80 is floated from the horizontal surface 100). In this case, for example, the electronic device 1 may start measurement of biological information upon being pressed by the subject in a direction indicated by an arrow P illustrated in Fig. 7 with the right hand or the like.

As illustrated in Fig. 7, the first abutment portion 11 may be brought into direct or indirect contact with the target region of the subject. As illustrated in Fig. 7, the second abutment portion 12 may be brought into direct or indirect contact with the vicinity of the region of the body where the first abutment portion 11 comes into contact with the target region of the subject. As illustrated in Fig. 7, a surface including the target region of the wrist of the subject typically has a curved shape. If the first abutment portion 11 and the second abutment portion 12 of the housing 10 have the same length in the Z-axis direction, the first abutment portion 11 may be floated from (the target region of) the wrist of the subject while the second abutment portion 12 comes into contact with the wrist of the subject. In one embodiment, accordingly, as illustrated in Fig. 7, the length of the first abutment portion 11 in the Z-axis direction may be longer than the length of the second abutment portion in the Z-axis direction. With this shape, the first abutment portion 11 can be appropriately brought into abutment against the target region of the subject while the second abutment portion 12 comes into contact with a portion of the wrist of the subject (for example, a portion S illustrated in Fig. 7).

In this manner, in one embodiment, the first abutment portion 11 may protrude from the housing 10 more than the second abutment portion 12 in the Z-axis direction illustrated in Fig. 7, for example. That is, the length by which the first abutment portion 11 protrudes from the housing 10 in the Z-axis positive direction may be larger than the length by which the second abutment portion 12 protrudes from the housing 10 in the Z-axis positive direction.

The shape of the first abutment portion 11 is not limited to the shape illustrated in Fig. 3 to Fig. 7, and may be any shape that enables the first abutment portion 11 to appropriately abut against the target region of the subject. Likewise, the shape of the second abutment portion 12 is not limited to the shape illustrated in Fig. 3 to Fig. 7, and may be any shape that enables the second abutment portion 12 to appropriately abut against a portion of the wrist of the subject (for example, the portion S illustrated in Fig. 7).

As illustrated in Fig. 7, the support portion 20 of the electronic device 1 may include the rear surface portion 22. The rear surface portion 22 may be a portion of the electronic device 1 that is pressed by the subject with a fingertip or the like. That is, by pressing the rear surface portion 22 with a fingertip or the like, the subject or the like can measure biological information using the electronic device 1 even if the base portion 80 or the support portion 20 is not pressed against the horizontal surface 100. As illustrated in Fig. 7, the rear surface portion 22 may be formed on the rear side of the support portion 20 (the surface directed to the Z-axis negative direction). In the example illustrated in Fig. 7, the rear surface portion 22 is formed in a location slightly above (in the Y-axis positive direction) the center of the support portion 20. However, the rear surface portion 22 may be formed in any location in accordance with the manner in which the electronic device 1 measures biological information, such that, for example, the rear surface portion 22 is formed substantially at the center of the support portion 20.

In the example illustrated in Fig. 7, furthermore, the rear surface portion 22 is illustrated as a shallow concave portion formed in the support portion 20. However, the shape of the rear surface portion 22 is not limited to a shallow concave portion. For example, the rear surface portion 22 may be formed as a shallow convex portion or the like formed in the support portion 20. Alternatively, the rear surface portion 22 may merely be, for example, a mark painted on the support portion 20 with paint or the like. In the electronic device 1, the rear surface portion 22 may be configured in any manner so long as it represents a portion to be pressed by the subject with a fingertip or the like.

In the electronic device 1, the first abutment portion 11 is brought into abutment against the target region such as the wrist of the subject, and the base portion 80 or the support portion 20 is pressed against the horizontal surface 100 by the subject with a fingertip or the like. As a result, the electronic device 1 is brought into the state illustrated in Fig. 1 or Fig. 7 during measurement of biological information. When the electronic device 1 is to be brought into abutment against the target region such as the wrist of the subject, the electronic device 1 may be positioned such that the first abutment portion 11 comes into abutment against the target region of the subject. At this time, as illustrated in Fig. 7, the electronic device 1 may be positioned such that, for example, the first abutment portion 11 comes into abutment against a region of the body where the ulnar artery or radial artery of the subject is present beneath the skin. That is, the target region for which the electronic device 1 according to an embodiment measures biological information of the subject may be, for example, a position where the radial artery or ulnar artery of the subject flows beneath the skin.

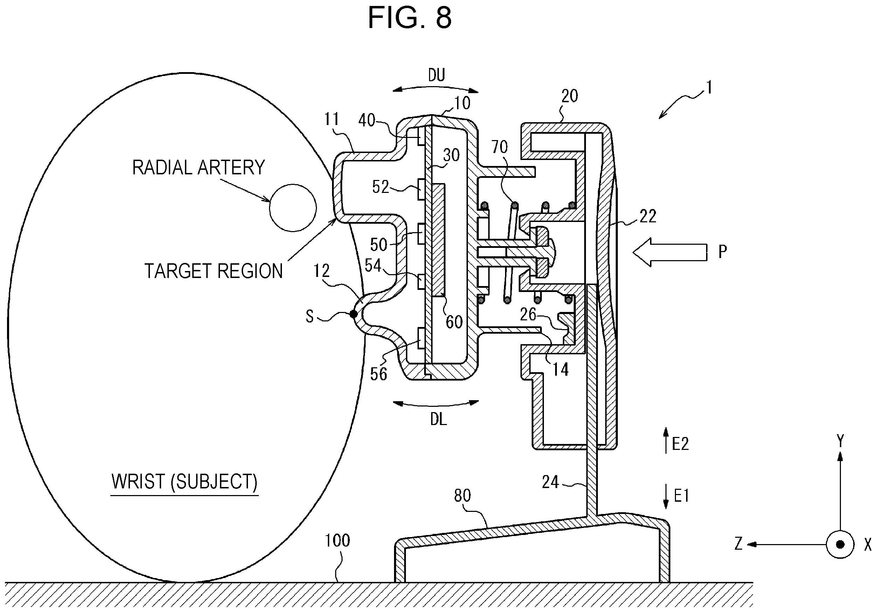

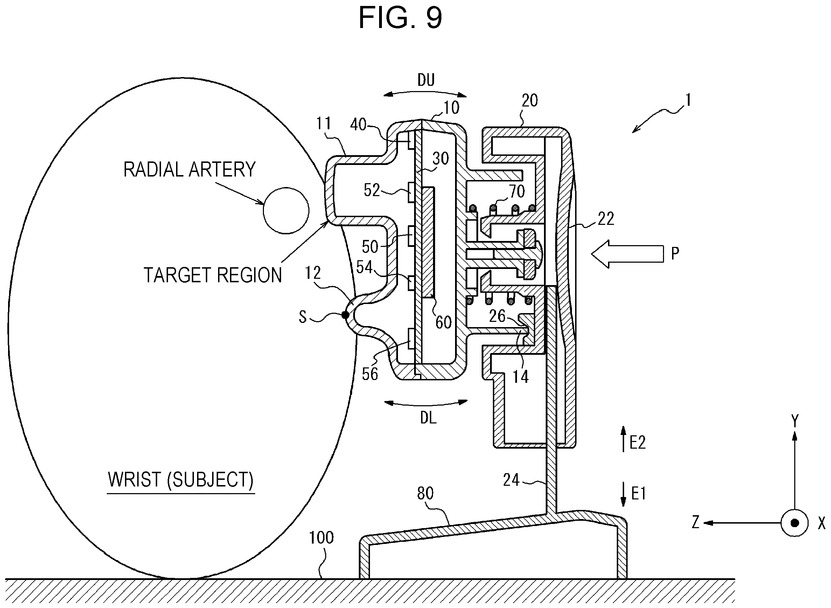

Fig. 8 and Fig. 9 are diagrams illustrating a cross section of the electronic device 1, together with a cross section of the wrist of the subject. Fig. 8 is a diagram illustrating a cross section of the electronic device 1 illustrated in Fig. 7, together with a cross section of the wrist of the subject. Fig. 9 is a sectional view illustrating a state in which the base portion 80 is pressed against (secured to) the horizontal surface 100 to apply a force to the support portion 20 of the electronic device 1 illustrated in Fig. 8 in the direction indicated by the arrow P illustrated in the drawing. The force in the direction indicated by the arrow P may be the reaction of a force with which the target region of the subject presses (the housing 10 of) the electronic device 1.

As illustrated in Fig. 8 and Fig. 9, the electronic device 1 includes, in appearance, the housing 10, the support portion 20, and the base portion 80. As described above, the housing 10 includes the first abutment portion 11 and the second abutment portion 12. The support portion 20 may include the rear surface portion 22 and the extension portion 24.

As illustrated in Fig. 8 and Fig. 9, the housing 10 of the electronic device 1 may further include a substrate 30. The substrate 30 may be a typical circuit board on which various electronic components and the like can be arranged. In one embodiment, the substrate 30 may be built in the housing 10 of the electronic device 1.

Various electronic components may be arranged on the surfaces of the substrate 30 on the Z-axis negative and positive direction sides. In the example illustrated in Fig. 8 and Fig. 9, a notification unit 40, the sensor 50, a control unit 52, a storage unit 54, and a communication unit 56 are arranged on the surfaces of the substrate 30 on the Z-axis negative and positive direction sides. The switch 13 described above may also be arranged on the substrate 30.

The notification unit 40 notifies the subject or the like of, for example, information such as a measurement result of biological information. The notification unit 40 may be, for example, a light-emitting unit such as a light-emitting diode (LED). Alternatively, the notification unit 40 may be a display device such as a liquid crystal display (LCD), an organic EL display (OELD: Organic Electro-Luminescence Display), or an inorganic EL display (IELD: Inorganic Electro-Luminescence Display). Such a display device employed as the notification unit 40 makes it possible to display, for example, relatively detailed information such as the state of glucose metabolism or lipid metabolism of the subject.

The notification unit 40 may notify the subject of not only information such as a measurement result of biological information but also, for example, information such as on/off of the power supply of the electronic device 1 or whether biological information is being measured. At this time, for example, the notification unit 40 may notify the subject of information such as on/off of the power supply of the electronic device 1 or whether biological information is being measured by a different type of light emission from that when notifying the subject of information such as a measurement result of biological information.

In one embodiment, the notification unit 40 may not necessarily be constituted by a light-emitting unit. For example, the notification unit 40 may be constituted by a sound output unit such as a speaker or a buzzer. In this case, the notification unit 40 may notify the subject or the like of, for example, information such as a measurement result of biological information via various sounds, voices, or the like.

In one embodiment, the notification unit 40 may be constituted by, for example, a tactile sensation providing unit such as a vibrator or a piezoelectric element. In this case, the notification unit 40 may notify the subject or the like of, for example, information such as a measurement result of biological information via various types of vibration, tactile sensation feedback, or the like.

The sensor 50 includes, for example, an angular speed sensor and detects pulsation from the target region to acquire a pulse wave. The sensor 50 may detect a displacement of the first abutment portion 11 (pulse contact portion) based on the pulse wave of the subject. The sensor 50 may be, for example, an acceleration sensor or may be a sensor such as a gyro sensor. Alternatively, the sensor 50 may be an angular speed sensor. The sensor 50 will further be described below.

As illustrated in Fig. 8 and Fig. 9, the sensor 50 is secured to the substrate 30. The substrate 30 is secured within the housing 10. The first abutment portion 11 is secured to the outside of the housing 10. Thus, a movement of the first abutment portion 11 is transmitted to the sensor 50 through the housing 10 and the substrate 30. Accordingly, the sensor 50 can detect the pulsation in the target region of the subject through the first abutment portion 11, the housing 10, and the substrate 30.

In the example illustrated in Fig. 8 and Fig. 9, the sensor 50 is arranged such that the sensor 50 is built in the housing 10. However, in one embodiment, the sensor 50 may not be entirely built in the housing 10. In one embodiment, the sensor 50 may be included in at least part of the housing 10. The sensor 50 may have any configuration in which a movement of at least one of the first abutment portion 11, the housing 10, and the substrate 30 is transmitted to the sensor 50.

The control unit 52 is a processor that controls and manages the entire electronic device 1, including the functional blocks of the electronic device 1. Further, the control unit 52 is a processor that calculates, from the acquired pulse wave, an index based on the propagation phenomenon of the pulse wave. The control unit 52 is constituted by a processor such as a CPU (Central Processing Unit) that executes a program specifying a control procedure and a program for calculating an index based on the propagation phenomenon of the pulse wave, and the programs are stored in a storage medium, such as the storage unit 54, for example. Further, the control unit 52 estimates a state related to glucose metabolism, lipid metabolism, or the like of the subject on the basis of the calculated index. The control unit 52 may send data to the notification unit 40.

The storage unit 54 stores programs and data. The storage unit 54 may include any non-transitory storage medium such as a semiconductor storage medium and a magnetic storage medium. The storage unit 54 may include a plurality of types of storage media. The storage unit 54 may include a combination of a portable storage medium, such as a memory card, an optical disk, or a magneto-optical disk, and a storage medium reading device. The storage unit 54 may include a storage device used as a temporary storage area such as a RAM (Random Access Memory). The storage unit 54 stores various types of information and/or programs for operating the electronic device 1, and also functions as a work memory. The storage unit 54 may store, for example, a measurement result of the pulse wave acquired by the sensor 50.

The communication unit 56 performs wired communication or wireless communication with an external device to transmit and receive various data. The communication unit 56 communicates with, for example, an external device that stores biological information of the subject to manage the health condition, and transmits the measurement result of the pulse wave measured by the electronic device 1 and/or the health condition estimated by the electronic device 1 to the external device. The communication unit 56 may be, for example, a communication module that supports Bluetooth (registered trademark), Wi-Fi, or the like.

As illustrated in Fig. 8 and Fig. 9, a battery 60 may be arranged on the surface of the substrate 30 on the Z-axis negative direction side. In this case, a battery holder may be arranged on the surface of the substrate 30 on the Z-axis negative direction side to secure the battery 60 in position. The battery 60 may be any power supply, for example, a button battery (coin battery) such as CR2032. Alternatively, the battery 60 may be, for example, a rechargeable storage battery. The battery 60 may include, for example, a lithium-ion battery and a control circuit or the like for charging and discharging the lithium-ion battery, if necessary. The battery 60 may supply power to the functional units of the electronic device 1.

The arrangement of the notification unit 40, the sensor 50, the control unit 52, the storage unit 54, the communication unit 56, and the battery 60 is not limited to that in the examples illustrated in Fig. 8 and Fig. 9. For example, the functional units described above may be arranged at any positions on the substrate 30. The functional units described above may be each arranged on either side of the substrate 30, as necessary. In a case where the electronic device 1 is connected to an external device in a wired or wireless manner, for example, at least some of the functional units such as the switch 13, the notification unit 40, the control unit 52, the storage unit 54, and the communication unit 56 may be included in the external device, as necessary.

As illustrated in Fig. 8 and Fig. 9, in the electronic device 1, the end of the housing 10 on the Z-axis negative direction side is connected to the end of the support portion 20 on the Z-axis positive direction side. As illustrated in Fig. 8 and Fig. 9, the housing 10 has, on the side thereof in the negative direction of the Z axis, a connection portion to be connected to the support portion 20. As illustrated in Fig. 8 and Fig. 9, the support portion 20 has, on the side thereof in the positive direction of the Z axis, an opening into which the connection portion of the housing 10 is inserted. In the example illustrated in Fig. 8 and Fig. 9, the connection portion of the housing 10 is configured to have a smaller size than the opening in the support portion 20 such that the connection portion of the housing 10 is inserted into the opening in the support portion 20. However, in one embodiment, the housing 10 may have an opening, and the support portion 20 may have an insertion portion. In this case, the opening in the housing 10 may be configured to have a larger size than the insertion portion of the support portion 20 such that the insertion portion of the support portion 20 is inserted into the opening in the housing 10. In both cases, the housing 10 and the support portion 20 may be configured to be movable freely to some extent without interfering with each other.

As illustrated in Fig. 8 and Fig. 9, in the electronic device 1, the housing 10 and the support portion 20 are connected to each other through an elastic member 70. In the examples illustrated in Fig. 8 and Fig. 9, the housing 10 and the support portion 20 are directly connected by the elastic member 70. However, for example, the elastic member 70 may indirectly connect the housing 10 and the support portion 20. For example, in one embodiment, the elastic member 70 may connect any member on the housing 10 side and any member on the support portion 20 side to each other. The elastic member 70 may be an elastic member deformable along at least any one axis among three axes orthogonal to one another (for example, the Y axis, the Y axis, and the Z axis). The elastic member 70 is a three-dimensionally deformable member.

Fig. 8 and Fig. 9 illustrate an example in which the elastic member 70 is a spring such as a compression coil spring. However, in one embodiment, the elastic member 70 may be constituted by, for example, any elastic body having appropriate elasticity, such as a spring, a resin, a sponge, or a silicone sheet, or may be any combination thereof. The elastic member 70 may be formed by, for example, a silicone sheet of a predetermined thickness having predetermined elasticity.

Fig. 8 illustrates a state in which no force (or a very weak force) is applied to the support portion 20 in the direction indicated by the arrow P. That is, Fig. 8 illustrates a state in which the support portion 20 is not (substantially) pressed toward the target region. In contrast, Fig. 9 illustrates a state in which a force is applied to the support portion 20 in the direction indicated by the arrow P. That is, Fig. 9 illustrates a state in which the support portion 20 is pressed toward the target region. Since such a pressing force deforms the elastic member 70, the length of the elastic member 70 illustrated in Fig. 9 in the Z-axis direction is shorter than the length of the elastic member 70 illustrated in Fig. 8 in the Z-axis direction. As described above, the force in the direction indicated by the arrow P illustrated in Fig. 8 or Fig. 9 may be a force generated in response to the base portion 80 or the support portion 20 being pressed against (secured to) the horizontal surface 100 by the subject or the like. Alternatively, the force in the direction indicated by the arrow P illustrated in Fig. 8 or Fig. 9 may be the reaction of a force with which the subject presses the target region against (the housing 10, the first abutment portion 11 or the second abutment portion 12, or the like of) the electronic device 1.

In the example illustrated in Fig. 8, the electronic device 1 includes a stopper mechanism to prevent the housing 10 and the support portion 20 from being displaced a distance of a predetermined length or longer. That is, the electronic device 1 illustrated in Fig. 8 includes a mechanism for preventing the housing 10 from being removed or falling off from the support portion 20 even in a state where the electronic device 1 is not pressed in the direction indicated by the arrow P illustrated in the drawing. Fig. 8 illustrates a state in which the distance between the housing 10 and the support portion 20 is fixed, with the restoration force of the elastic member 70 maintained to some extent. In this situation, the housing 10 and the support portion 20 are not displaced a larger distance.

In the situation illustrated in Fig. 8, if a pressing force is applied in the direction indicated by the arrow P illustrated in the drawing, in contrast, as illustrated in Fig. 9, the elastic member 70 is deformed so as to contract. In the situation illustrated in Fig. 9, a protruding portion 14 of the housing 10 reaches and comes into contact with a receiving portion 26 of the support portion 20. If the pressing force in the direction indicated by the arrow P illustrated in the drawing becomes weaker than that in this state, a state can be implemented in which the protruding portion 14 of the housing 10 does not come into contact with the receiving portion 26 of the support portion 20 while the elastic member 70 is somewhat contracted. In this state, the housing 10 can be displaced freely to some extent with respect to the support portion 20 connected through the elastic member 70. Accordingly, the electronic device 1 can satisfactorily detect pulsation in the target region of the subject.

In Fig. 8 and Fig. 9, the elastic member 70 is a spring such as a compression coil spring. However, as described above, for example, the elastic member 70 may be constituted by a silicone seed or the like of a predetermined thickness. In this case, the housing 10 and the support portion 20 may be bonded to the elastic member 70 with an adhesive, a double-sided adhesive tape, or the like. The elastic member 70 may be bonded to any other member such that the influence on the deformation of the elastic member 70 can be reduced. That is, the elastic member 70 may be configured to be appropriately deformable even when the elastic member 70 is bonded to any other member.

As described above, the electronic device 1 according to an embodiment includes the housing 10, the support portion 20, the sensor 50, the elastic member 70, and the base portion 80. The housing 10 includes, at least in part, the sensor 50. The sensor 50 is configured to be capable of detecting pulsation in a target region of a subject. The support portion 20 is configured to support the housing 10 on a side of the support portion 20. The elastic member 70 is interposed between the housing 10 and the support portion 20. The base portion 80 is configured to allow the support portion 20 to stand upright. The base portion 80 may allow the support portion 20 to stand upright such that the electronic device 1 is erected on a horizontal surface in a free-standing manner.

As illustrated in Fig. 8 and Fig. 9, in a state where the support portion 20 of the electronic device 1 is caused to stand upright by the base portion 80, the first abutment portion 11 of the housing 10 can come into contact with the target region of the subject, that is, the skin over the radial artery of the subject. As illustrated in Fig. 8 and Fig. 9, the housing 10 is supported on a side of the support portion 20. When the base portion 80 or the support portion 20 is pressed against the horizontal surface 100 by the subject or the like, the positions of the base portion 80 and the support portion 20 on the horizontal surface 100 are fixed. In this state, in response to the subject pressing the target region against the housing 10 (alternatively, the first abutment portion 11 or the second abutment portion 12, or the like), the base portion 80 and the support portion 20 generate a reaction of the force toward the target region, that is, in the direction indicated by the arrow P. Due to the elastic force of the elastic body 140 arranged between the support portion 20 to which a force is applied in the direction indicated by the arrow P and the housing 10 including the sensor 50, the sensor 50 is urged toward the target region of the subject (together with the housing 10 and the first abutment portion 11). The first abutment portion 11, which is urged by the elastic force of the elastic member 70, comes into contact with the skin over the radial artery of the subject. In this case, the first abutment portion 11 is displaced in accordance with the movement of the radial artery of the subject, that is, the pulsation. Accordingly, the sensor 50, which operates in association with the first abutment portion 11, is also displaced in accordance with the movement of the radial artery of the subject, that is, the pulsation. For example, as illustrated in Fig. 8 and Fig. 9, in a state where a force is applied to the support portion 20 in the direction indicated by the arrow P, the housing 10 can be displaced about an axis S in a direction indicated by an arrow DU or an arrow DL. The axis S may be a portion of the second abutment portion 12 of the housing 10 that contacts the wrist of the subject.

In this embodiment, the sensor 50, which operates in association with the first abutment portion 11, is coupled to the support portion 20 through the elastic member 70. Thus, the sensor 50 is given a somewhat free range of motion because of the flexibility of the elastic member 70. The flexibility of the elastic member 70 further makes it difficult to hinder the movement of the sensor 50. The elastic member 70 having appropriate elasticity deforms in accordance with the pulsation in the target region of the subject. In the electronic device 1 according to this embodiment, therefore, the sensor 50 can sensitively detect the pulsation in the target region of the subject. In addition, the electronic device 1 according to this embodiment is displaced in accordance with the pulse wave, which can eliminate the congestion of the subject and eliminate the pain of the subject. In this manner, in this embodiment, the elastic member 70 may be deformable in accordance with the pulsation in the target region of the subject. Further, the elastic member 70 may be elastically deformed to such an extent that the pulsation in the target region of the subject is detectable by the sensor 50.

As described above, the electronic device 1 according to an embodiment can function as a small and lightweight measurement device. The electronic device 1 according to an embodiment is not only excellent in portability but also capable of extremely easily measuring biological information of the subject. In addition, the electronic device 1 according to an embodiment can maintain a free-standing posture before measurement or the like. This allows the subject to easily position the target region when bringing the target region into abutment against the first abutment portion 11. Further, in the electronic device 1 according to an embodiment, it is sufficient that the base portion 80 or the support portion 20 be pressed downward during measurement. This eliminates the need for the subject to perform fine adjustment of the force pressing the base portion 80 or the support portion 20 during measurement. The electronic device 1 according to an embodiment can therefore provide relatively stable measurement of biological information of the subject. The electronic device 1 according to an embodiment can further measure the biological information alone, without cooperating with any other external device or the like. In this case, there is no need to carry any other accessory such as a cable. The electronic device 1 according to an embodiment can therefore increase usability.

In one embodiment, the electronic device 1 may include a mechanism such as a stopper between the housing 10 and the support portion 20. In Fig. 8 and Fig. 9, as an example, a configuration is illustrated in which the housing 10 includes the protruding portion 14 and the support portion 20 includes the receiving portion 26. That is, the housing 10 includes, as part of the connection portion to be connected to the support portion 20, the protruding portion 14. The support portion 20 includes, as part of the opening into which the connection portion of the housing 10 is inserted, the receiving portion 26, which can receive the protruding portion 14. In the following, the protruding portion 14 and the receiving portion 26 are collectively referred to also as a "stopper (14, 26)".

As illustrated in Fig. 8 and Fig. 9, the stopper (14, 26) is formed only in a portion of the insertion portion of the housing 10 and the opening in the support portion 20 where the housing 10 and the support portion 20 are connected to each other. For example, in the examples illustrated in Fig. 8 and Fig. 9, the stopper (14, 26) is formed only at the lower end of a portion where the housing 10 and the support portion 20 are connected to each other. In contrast, the stopper (14, 26) is not formed at the upper end or the like of the portion where portion where the housing 10 and the support portion 20 are connected to each other. In one embodiment, the stopper (14, 26) may not be formed at the upper end of the portion where the housing 10 and the support portion 20 are connected to each other or a portion other than the lower end of the portion where the housing 10 and the support portion 20 are connected to each other.

As described above, the stopper (14, 26), which is provided only in a portion, makes it difficult to suppress the movement of the housing 10 relative to the support portion 20 even when the subject relatively strongly presses the target region against the support portion 20. For example, in the situation illustrated in Fig. 8, the subject does not strongly press the target region against the support portion 20, and thus the protruding portion 14 and the receiving portion 26 do not abut against each other. In the situation illustrated in Fig. 9, in contrast, the subject strongly presses the target region against the support portion 20. Thus, the elastic member 70 is deformed, which causes a displacement of the housing 10 relative to the support portion 20. As a result, the protruding portion 14 and the receiving portion 26 abut against each other. Even in this case, the housing 10 and the support portion 20 do not abut against each other in a portion other than the portion where the protruding portion 14 and the receiving portion 26 abut against each other. Thus, as the movement of the housing 10 relative to the support portion 20, the movement indicated by the arrow UL illustrated in the drawing is not substantially suppressed although the movement indicated by the arrow DL illustrated in the drawing is somewhat suppressed. It is therefore difficult to suppress the movement of the housing 10 relative to the support portion 20 even when the subject relatively strongly presses the target region against the support portion 20.

Fig. 8 and Fig. 9 present a configuration in which the housing 10 includes the protruding portion 14 and the support portion 20 includes the receiving portion 26. However, these may be reversed. That is, in one embodiment, the housing 10 may include the receiving portion 26, and the support portion 20 may include the protruding portion 14.

In this manner, the electronic device 1 according to an embodiment may include the stopper (14, 26). The stopper (14, 26) may include the protruding portion 14 and the receiving portion 26. The protruding portion 14 may be formed in one of the housing 10 and the support portion 20. The receiving portion 26 may be formed in the other of the housing 10 and the support portion 20. The stopper (14, 26) may be configured such that the protruding portion 14 is receivable in the receiving portion 26. In one embodiment, the stopper (14, 26) may be configured to allow the housing 10 to partially abut against the support portion 20 in response to the housing 10 being displaced with respect to the support portion 20 due to a deformation of the elastic member 70.

In this embodiment, the sensor 50 may be, for example, a sensor that detects, for each of a plurality of axes, at least one of the angle (inclination), angular speed, and angular acceleration of an object, such as a gyro sensor (gyroscope). In this case, the sensor 50 can detect complex motion based on the pulsation in the target region of the subject as the respective parameters for the plurality of axes. Alternatively, the sensor 50 may be a six-axis sensor that is a combination of a three-axis gyro sensor and a three-axis acceleration sensor.

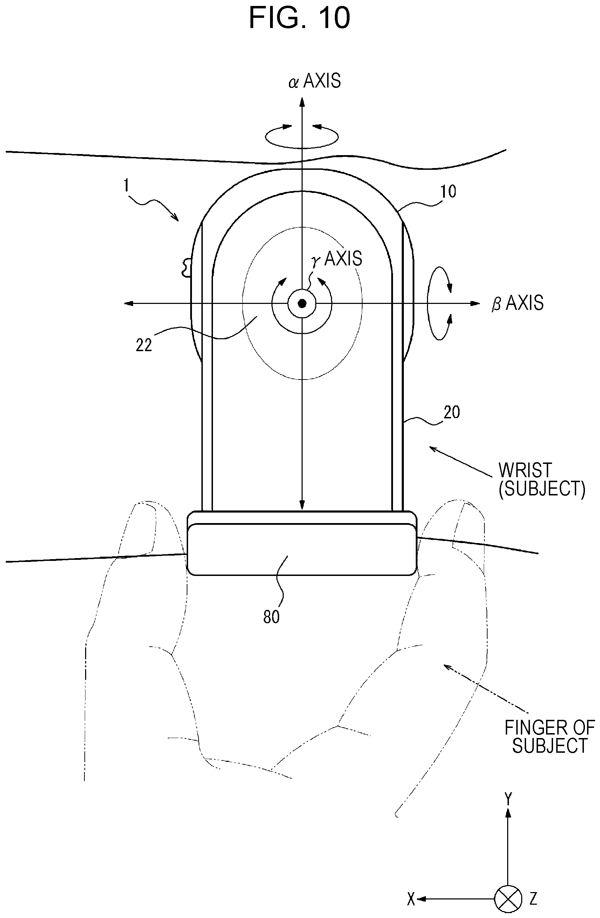

Fig. 10 is a diagram illustrating an example manner of using the electronic device 1. Fig. 10 is a diagram illustrating an enlarged version of the situation illustrated in Fig. 1 when viewed from a different viewpoint.

For example, as illustrated in Fig. 10, the sensor 50 built in the housing 10 of the electronic device 1 may detect a rotational movement about each of three axes, namely an α axis, a β axis, and a γ axis. The α axis may be, for example, an axis extending in a direction substantially orthogonal to the radial artery of the subject. The β axis may be, for example, an axis extending in a direction substantially parallel to the radial artery of the subject. The γ axis may be, for example, an axis extending in a direction substantially orthogonal to both the α axis and the β axis.

In this embodiment, accordingly, the sensor 50 may detect pulsation in the target region of the subject as a portion of a rotational movement about a predetermined axis. Alternatively, the sensor 50 may detect pulsation in the target region of the subject as rotational movements on at least two axes or as rotational movements on three axes. In the present disclosure, the "rotational movement" may not necessarily be a movement including a displacement along a circular orbit by one or more turns. For example, in the present disclosure, the rotational movement may be, for example, a partial displacement along a circular orbit by less than one turn (for example, a displacement along an arc) .

As illustrated in Fig. 10, the electronic device 1 according to this embodiment can detect, for example, respective rotational movements about three axes using the sensor 50. The electronic device 1 according to this embodiment combines the plurality of results detected by the sensor 50 by, for example, adding them up, and can thus increase the detection sensitivity of the pulse wave of the subject. The computation, such as adding up, may be performed by the control unit 52, for example. In this case, the control unit 52 may calculate the index of the pulse wave based on the pulsation detected by the sensor 50.

For example, in the example illustrated in Fig. 10, the changes in signal strength with time based on the rotational movements of the sensor 50 about the α axis and the β axis have remarkable peaks based on the pulse wave of the subject. Thus, for example, the control unit 52 adds up the detection results for the α axis, the β axis, and the γ axis, and can thus increase the detection accuracy of the pulse wave of the subject. The electronic device 1 according to this embodiment can therefore improve the usefulness when the subject measures the pulse wave.

In one embodiment, the control unit 52 of the electronic device 1 may calculate the index of the pulse wave based on the pulsation detected by the sensor 50. In this case, the control unit 52 may combine (for example, add up) the results detected by the sensor 50 as rotational movements on at least two axes (for example, rotational movements on three axes). The electronic device 1 according to this embodiment can detect pulse wave signals of a plurality of directions. Thus, the electronic device 1 according to this embodiment combines detection results for a plurality of axes, thereby increasing the signal strength compared to a detection result for a single axis. The electronic device 1 according to this embodiment can therefore detect a signal having a good SN ratio and increase the detection sensitivity, making it possible to achieve stable measurement.

In the detection result for the γ axis illustrated in Fig. 10, the peak based on the pulse wave of the subject is expected not to appear more noticeably than that in the detection result for the remaining α axis or β axis. In this manner, adding a detection result having a low signal level, such as the detection result for the γ axis, to a detection result for another axis may result in a reduction in SN ratio. In addition, a detection result having a low signal level may be mostly regarded as a noise component. In this case, the detection result having a low signal level may not contain a satisfactory pulse wave component. In this embodiment, accordingly, if there is an axis for which the detection result is less than a predetermined threshold among the detection results for the plurality of axes, the control unit 52 may not add the detection result for the axis.

For example, it is assumed that the pulsation of a certain subject is detected by the sensor 50 as the respective rotational movements about the α axis, the β axis, and the γ axis. As a result, the peak values in the detection results for the α axis, the β axis, and the γ axis are assumed to exceed the predetermined threshold. In this case, the control unit 52 may add up all of the detection result for the α axis, the detection result for the β axis, and the detection result for the γ axis to calculate the sum as the index of the pulse wave based on the pulsation detected by the sensor 50.

On the other hand, for example, as a result of detecting the pulsation of a certain subject, the peak values in the detection results for the α axis and the β axis are assumed to exceed the predetermined threshold. However, the peak value in the detection result for the γ axis is assumed not to exceed the predetermined threshold. In this case, the control unit 52 may add up only the detection results for the α axis and the β axis to calculate the sum as the index of the pulse wave based on the pulsation detected by the sensor 50.

When performing such processing, the control unit 52 may set thresholds, which are used as a reference to determine whether the detection results for the respective axes are to be added up, to be separate for the respective axes or to be the same for the respective axes. In both cases, a threshold may be set appropriately so that the pulsation of the subject can be suitably detected in a detection result for each axis.

In this manner, in the electronic device 1 according to this embodiment, the control unit 52 may combine only results having components greater than or equal to a predetermined threshold among the results detected by the sensor 50 as rotational movements on at least two axes. Thus, the electronic device 1 according to this embodiment can suppress the reduction in the SN ratio of a detection result. The electronic device 1 according to this embodiment can therefore improve the usefulness when the subject measures the pulse wave.

As described above, when adding up detection results for a plurality of axes, merely adding up the detection results for the respective axes may cause a problem. This is presumably because the results detected by the sensor 50 do not match in polarity depending on the positional relationship between the direction of the pulsation of the subject and the sensor 50. For example, a detection result for a certain axis may be reversed in polarity between when the pulsation of the right hand of the subject is detected and when the pulsation of the left hand of the subject is detected using the sensor 50.

For example, when the pulsation of the subject is detected, it is assumed that an upward peak is approximately periodically detected for a detection result for a certain axis. However, it is also assumed that a downward peak is approximately periodically detected for a detection result for another axis. In this manner, when detection results for a plurality of axes are reversed in polarity, merely adding up the detection results may cause the peaks to be canceled out each other, and a satisfactory result may not be obtained.

In this embodiment, accordingly, when detection results for a plurality of axes are reversed in polarity, the control unit 52 may invert the polarity of the detection result for at least one axis before adding the detection result to the detection results for the other axes. For example, if detection results for two axes are reversed in polarity, the control unit 52 may invert the polarity of the detection result for one axis in accordance with the other axis.

In this manner, in the electronic device 1 according to this embodiment, the control unit 52 may combine the results detected by the sensor 50 as rotational movements on at least two axes after the polarities of the results are made to match each other. The electronic device 1 according to this embodiment can increase the detection accuracy of the pulse wave of the subject. The electronic device 1 according to this embodiment can therefore improve the usefulness when the subject measures the pulse wave.

As described above, when processing for matching the polarities of detection results for a plurality of axes is performed by inverting the polarity of the detection result for at least one axis, it is necessary to determine the directions of the polarities in the respective detection results. The determination of the polarity directions can be performed by various methods. For example, the control unit 52 may determine whether the peak of the detection result for each axis is directed to the positive direction side or the negative direction side of the signal strength. Alternatively, for example, the control unit 52 may determine whether the peak of the detection result for each axis is larger or smaller than the average value of the signal. In order to invert the polarity of the detection result for at least one axis, the control unit 52 may multiply the detection result whose polarity is to be inverted by minus 1.

Further, after appropriately inverting the polarity of a detection result in the way described above, the control unit 52 may add or subtract a predetermined value to or from the entire detection result and then add the detection result to the detection results for the other axes. Alternatively, before adding up the detection results for the plurality of axes, the control unit 52 may appropriately weight the detection results for the respective axes or appropriately correct the detection results for the respective axes.

Next, the electronic device 1 according to some embodiments will be described.

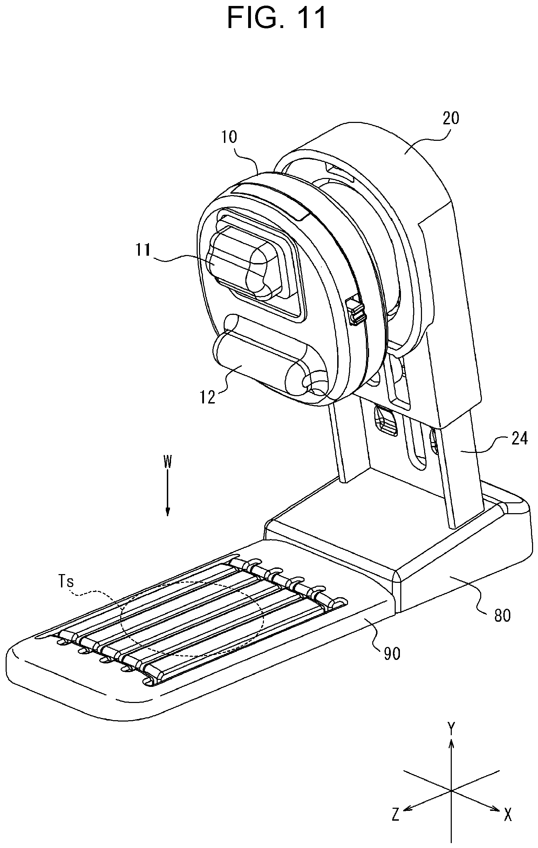

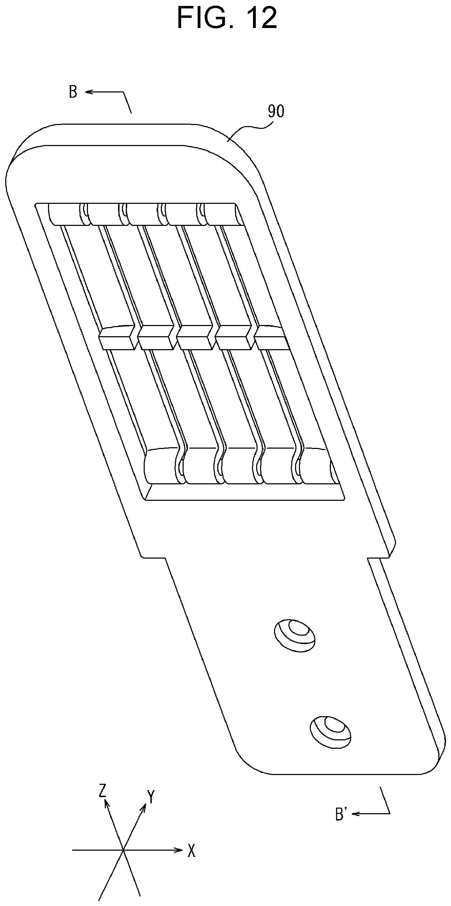

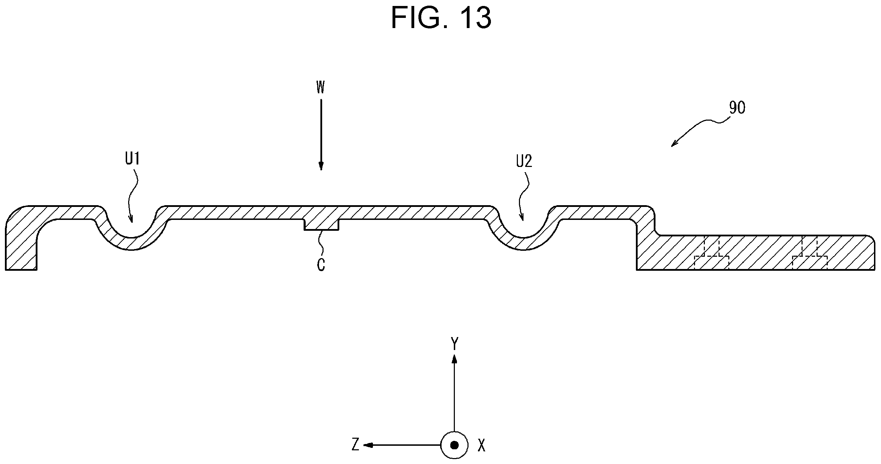

Fig. 11 is a perspective view illustrating the electronic device 1 according to an embodiment. The electronic device 1 illustrated in Fig. 11 is configured by modifying the electronic device 1 illustrated in Fig. 3 to Fig. 6 described above such that the base portion 80 includes a wrist rest portion 90. Fig. 12 is a perspective view of only the wrist rest portion 90 illustrated in Fig. 11 from a different viewpoint from that in Fig. 11. Fig. 12 illustrates the wrist rest portion 90 illustrated in Fig. 11 from a viewpoint on the bottom surface side. Fig. 13 is a sectional view of the wrist rest portion 90 illustrated in Fig. 12, taken along line B-B'.

As illustrated in Fig. 11, the base portion 80 of the electronic device 1 may include the wrist rest portion 90. The wrist portion including the target region of the subject may be placed on the wrist rest portion 90 when the subject measures biological information. For example, when measuring biological information, the subject may place their wrist portion including the target region in an area Ts illustrated in Fig. 11.

The wrist rest portion 90 may be made of, for example, plastic and may be made of a material having appropriate rigidity, such as resin. The wrist rest portion 90 may be made of a material and have a shape such that the wrist rest portion 90 is not easily damaged even when the wrist portion including the target region of the subject is placed on the wrist rest portion 90. The shape of the wrist rest portion 90 is not limited to the shape illustrated in Fig. 11 to Fig. 13, and may be various shapes in terms of functionality of a measurement device and/or design viewpoint or the like.

As illustrated in Fig. 11 and Fig. 12, at least a portion of the wrist rest portion 90 may be formed with slits. As illustrated in Fig. 11, the wrist rest portion 90 may be formed with slits in or around the area Ts. The formation of such slits allows the subject to easily place their wrist portion including the target region in or around the area Ts. Upon the wrist portion of the subject being placed in or around the area Ts, the portion of the wrist rest portion 90, which is formed with slits, bends appropriately. Thus, the wrist rest portion 90 having slits can reduce the pain or burden that the subject feels when, for example, the ulnar styloid process in the wrist of the subject is brought into abutment against around the area Ts.

As illustrated in Fig. 13, for example, the wrist rest portion 90 may have bends, such as U-shaped bends, around both ends of the slits formed in the wrist rest portion 90. The wrist rest portion 90 illustrated in Fig. 13 has a bend U1 and a bend U2 at both ends of the slit. By forming such bends, it is possible to implement adjustment of bends such as increasing the number of bends in the wrist rest portion 90 (in particular, in or around the area Ts).

As illustrated in Fig. 12 and Fig. 13, furthermore, convex portions C may be formed around, for example, a center portion on the back side (bottom surface side) of the surface of the wrist rest portion 90 that is formed with slits. As illustrated in Fig. 13, when the slit in the wrist rest portion 90 is pressed and bent in a direction indicated by an arrow W illustrated in the drawing, the convex portion C abuts against the horizontal surface 100 before the slit is completely bent. The formation of the convex portions C can reduce the risk of damage caused by excessive bending of the slits in the wrist rest portion 90 when, for example, the subject relatively strongly places their wrist portion on the wrist rest portion 90.

In Fig. 11 to Fig. 13, the wrist rest portion 90 has been described as being attachable to the base portion 80 of the electronic device 1, as an example. In this case, the wrist rest portion 90 may be attached to the base portion 80 of the electronic device 1 by using, for example, screws or the like or may be bonded to the base portion 80 by using an adhesive or the like. For example, the wrist rest portion 90 may be configured to be removable from the base portion 80 of the electronic device 1, if necessary. Alternatively, the wrist rest portion 90 may be formed integrally with, for example, the base portion 80 and may be configured not to be removable from the base portion 80.

In this manner, the base portion 80 may include the wrist rest portion 90 on which the wrist portion including the target region of the subject is placeable. In this case, a portion of the wrist rest portion 90 where the wrist portion of the subject is to be placed may be configured to have elasticity. The portion of the wrist rest portion 90 where the wrist portion of the subject is to be placed may be formed with slits.

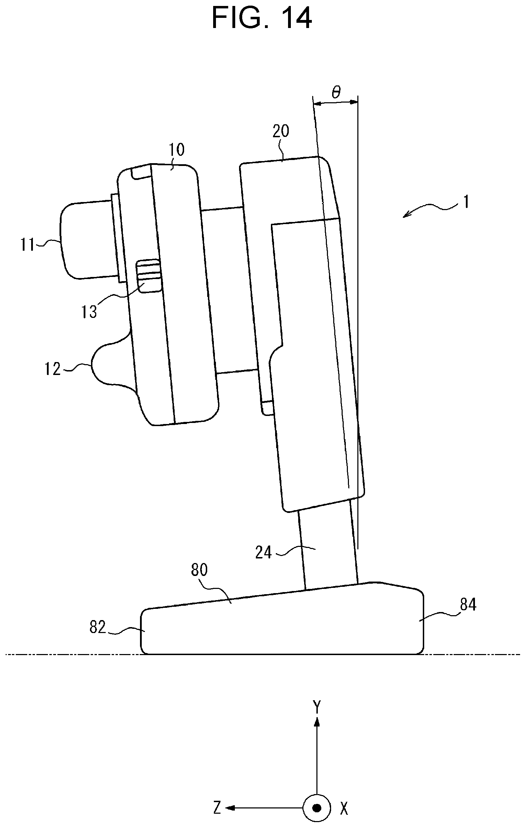

Fig. 14 is a diagram illustrating the appearance of the electronic device 1 according to an embodiment. The electronic device 1 illustrated in Fig. 14 is configured by changing the angle of the support portion 20 in the electronic device 1 illustrated in Fig. 4.

In the electronic device 1 illustrated in Fig. 4, the support portion 20 is coupled to the base portion 80 so as to be directed to the positive direction of the Y axis, that is, the direction normal to the horizontal surface 100. In the electronic device 1 depicted in Fig. 14, in contrast, the support portion 20 is coupled to the base portion 80 so as to be directed to a direction inclined by an angle θ from the positive direction of the Y axis (the direction normal to the horizontal surface 100). In the electronic device 1 according to an embodiment, accordingly, the support portion 20 may be coupled to the base portion 80 at an angle that makes it easy for the subject to measure biological information at the target region. In Fig. 14, the angle θ at which the support portion 20 is attached may be an angle at which the support portion 20 is inclined to the positive direction of the Z axis from the positive direction of the Y axis (the direction normal to the horizontal surface 100), or an angle at which the support portion 20 is inclined to the negative direction of the Z axis from the positive direction of the Y axis (the direction normal to the horizontal surface 100). In the following, the angle θ illustrated in Fig. 14 is also referred to as an "attachment angle θ", as necessary.

As described above, the base portion 80 may allow the support portion 20 to stand upright such that the electronic device 1 is erected on the horizontal surface 100 in a free-standing manner. If the attachment angle θ illustrated in Fig. 14 is increased to some extent, the electronic device 1 may be unstably erected in a free-standing manner, or the electronic device 1 may be no longer erected in a free-standing manner. Accordingly, for example, the shape of the bottom surface of the base portion 80 may be appropriately changed to adjust the balance to keep the electronic device 1 erected in a free-standing manner. For example, in Fig. 14, if the attachment angle θ is increased to some extent, it is predicted that the electronic device 1 may be inclined or fall down in the positive direction of the Z axis. However, the base portion 80 is formed into a shape such that a front portion 82 thereof is extended, thereby making it possible to adjust the balance to keep the electronic device 1 erected in a free-standing manner. In this case, the bottom surface portion of the base portion 80 may have a shape extending in the positive direction of the Z axis. In this case, furthermore, a rear portion 84 of the base portion 80 may be shortened with the extension of the front portion 82 of the base portion 80. That is, the bottom surface portion of the base portion 80 may have a shape that is shortened in the negative direction of the Z axis.

Instead of or in addition to such a change in the shape of the bottom surface portion of the base portion 80 as described above, the position of the center of gravity of the entire electronic device 1 may be adjusted. For example, when the attachment angle θ illustrated in Fig. 14 is to be increased to some extent, the masses of the housing 10 and the built-in components may be reduced. In this case, the masses of at least one of the support portion 20 and the base portion 80 and the built-in components may be increased. An increase in the attachment angle θ illustrated in Fig. 14 to some extent shifts the center of gravity of the entire electronic device 1 toward the positive direction of the Z axis. In this case, the masses of the components forming the electronic device 1 may be adjusted so that the center of gravity of the entire electronic device 1 can be shifted toward the negative direction of the Z axis.

In this manner, the bottom surface portion of the base portion 80 may be formed into a shape such that the electronic device 1 is erected on a horizontal surface in a free-standing manner. The center of gravity of the electronic device 1 may be positioned so that the electronic device 1 is erected on a horizontal surface in a free-standing manner.

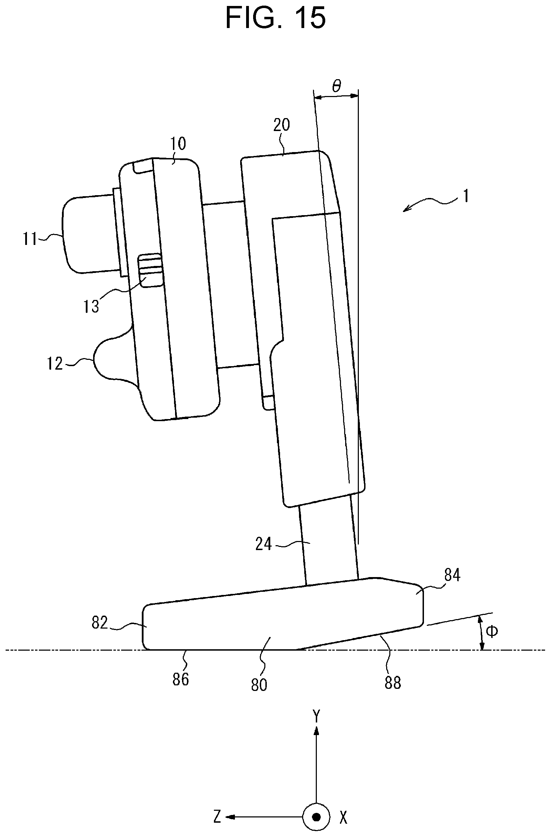

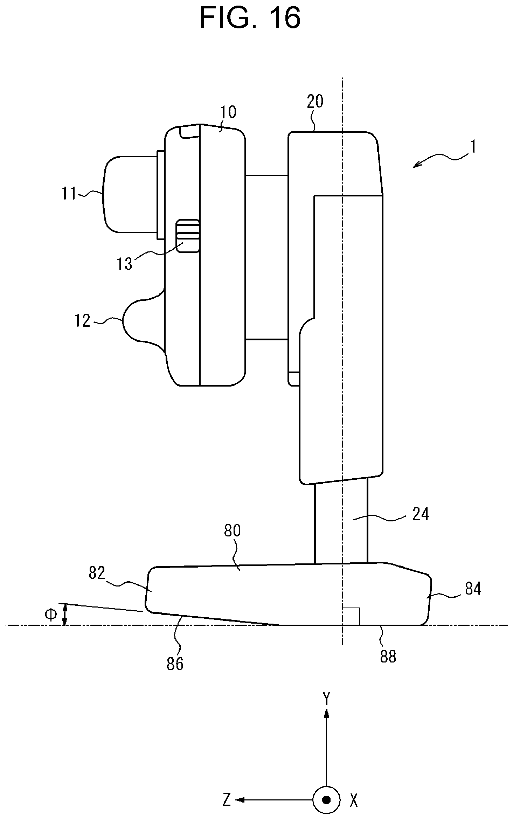

Fig. 15 and Fig. 16 are diagrams illustrating the appearance of the electronic device 1 according to an embodiment. The electronic device 1 illustrated in Fig. 15 and Fig. 16 is configured by changing the bottom surface portion of the base portion 80 in the electronic device 1 illustrated in Fig. 14.

In the electronic device 1 illustrated in Fig. 14, the bottom surface portion of the base portion 80 has been described as being formed in a planar shape. In contrast, in the electronic device 1 illustrated in Fig. 15 and Fig. 16, the bottom surface portion of the base portion 80 has two flat surfaces. The two flat surfaces are formed so as to have different inclination angles. That is, as illustrated in Fig. 15 and Fig. 16, the electronic device 1 may be configured such that the bottom surface portion of the base portion 80 has two or more flat surfaces having different inclination angles. With this configuration, the angle of the support portion 20 at which the electronic device 1 is erected on the horizontal surface 100 in a free-standing manner can be changed. For example, in the electronic device 1 illustrated in Fig. 15, like the electronic device 1 illustrated in Fig. 14, the support portion 20 is directed to have an angle θ relative to the positive direction of the Y axis (the direction normal to the horizontal surface 100). In contrast, in the electronic device 1 illustrated in Fig. 16, like the electronic device 1 illustrated in Fig. 4, the support portion 20 is directed to the positive direction of the Y axis (the direction normal to the horizontal surface 100).

The electronic device 1 illustrated in Fig. 15 and Fig. 16 may be configured to be erected on the horizontal surface 100 in a free-standing manner in either of the states illustrated in Fig. 15 and Fig. 16. For example, in the electronic device 1, as described above, the bottom surface portion of the base portion 80 and/or the center of gravity of the electronic device 1 may be appropriately adjusted to erect the electronic device 1 in a free-standing manner in the state illustrated in Fig. 15 or the state illustrated in Fig. 16, or the electronic device 1 illustrated in Fig. 15 and Fig. 16 may be configured to be erected on the horizontal surface 100 in a free-standing manner in both the states illustrated in Fig. 15 and Fig. 16. For example, in the electronic device 1, as described above, the bottom surface portion of the base portion 80 and/or the center of gravity of the electronic device 1 may be appropriately adjusted to erect the electronic device 1 in a free-standing manner in both the state illustrated in Fig. 15 and the state illustrated in Fig. 16. For example, the center of gravity of the entire electronic device 1 may be adjusted to be balanced at an intermediate state between the state illustrated in Fig. 15 and the state illustrated in Fig. 16, thereby enabling the electronic device 1 to be erected in a free-standing manner in both the state illustrated in Fig. 15 and the state illustrated in Fig. 16.

In this manner, in the electronic device 1 according to an embodiment, the bottom surface portion of the base portion 80 may have two or more flat surfaces having different inclination angles. In this case, the bottom surface portion of the base portion 80 may be formed into a shape such that the electronic device 1 is erected in a free-standing manner with at least one of the two or more flat surfaces having different inclination angles coming into contact with the horizontal surface. The center of gravity of the electronic device 1 may be positioned such that the electronic device 1 is erected in a free-standing manner with at least one of the two or more flat surfaces having different inclination angles coming into contact with the horizontal surface.

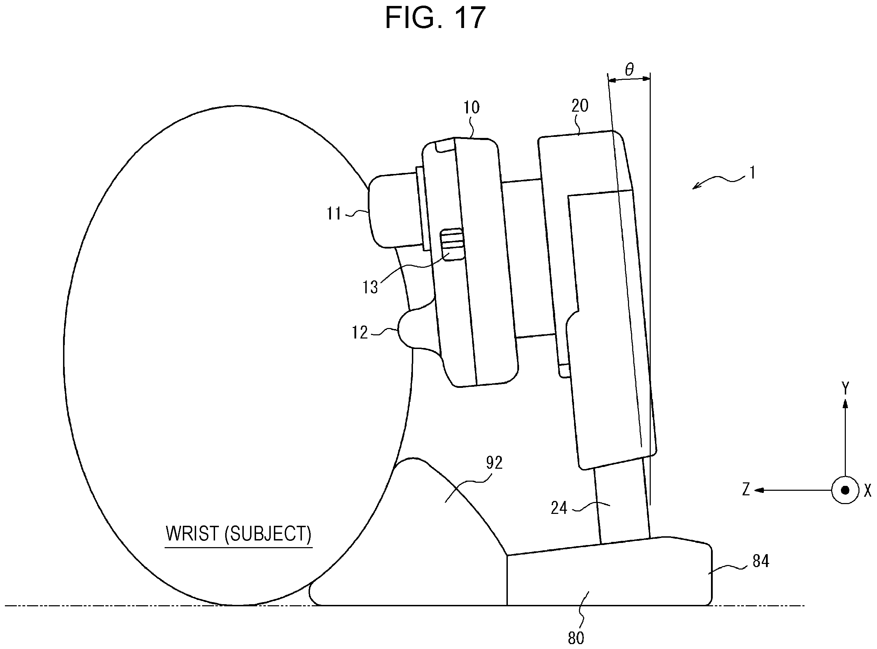

Fig. 17 is a diagram illustrating the electronic device 1 according to an embodiment. The electronic device 1 illustrated in Fig. 17 is configured by modifying the electronic device 1 illustrated in Fig. 7 such that the base portion 80 includes a wrist abutment portion 92. In the electronic device 1 illustrated in Fig. 17, like the electronic device 1 illustrated in Fig. 14, the angle of the support portion 20 is also changed.

As illustrated in Fig. 17, the base portion 80 of the electronic device 1 may include the wrist abutment portion 92. The wrist portion including the target region of the subject may be brought into abutment against the wrist abutment portion 92 when the subject measures biological information. For example, when measuring biological information, the subject may bring their wrist portion including the target region into abutment against the wrist abutment portion 92 of the base portion 80 illustrated in Fig. 17.

The wrist abutment portion 92 may be made of, for example, plastic and may be made of a material having appropriate rigidity, such as resin. The wrist abutment portion 92, against which the wrist portion of the subject is brought into abutment, may be configured to appropriately include, at least in part, for example, an elastic member such as a rubber or urethane member. The wrist abutment portion 92 may be made of a material and have a shape such that the wrist abutment portion 92 is not easily damaged even when the wrist portion including the target region of the subject is brought into abutment against the wrist abutment portion 92. The shape of the wrist abutment portion 92 is not limited to the shape illustrated in Fig. 17, and may be various shapes in terms of functionality for bringing the wrist portion of the subject into abutment and/or design viewpoint or the like.

When measuring biological information using the electronic device 1 illustrated in Fig. 17, the subject may first press and fix the base portion 80 or the support portion 20 of the electronic device 1, which is erected in a free-standing manner, with their finger or the like. Then, when positioning the target region to be measured on the housing 10 of the electronic device 1 (or the first abutment portion 11 or the like), the subject may bring their wrist portion into abutment against the wrist abutment portion 92. In this case, the relative positional relationship between the wrist abutment portion 92 and the first abutment portion 11 may be adjusted in advance for each individual subject. Accordingly, the subject can achieve measurement in the same environment each time measurement is performed, and is only required to bring their wrist into abutment against the wrist abutment portion 92 to immediately position the first abutment portion 11 with respect to the target region to be measured and start measurement of biological information.

In Fig. 17, the wrist abutment portion 92 has been described as being attachable to the base portion 80 of the electronic device 1, as an example. In this case, the wrist abutment portion 92 may be attached to the base portion 80 of the electronic device 1 by using, for example, screws or the like or may be bonded to the base portion 80 by using an adhesive or the like. For example, the wrist abutment portion 92 may be configured to be removable from the base portion 80 of the electronic device 1, if necessary. Alternatively, the wrist abutment portion 92 may be formed integrally with, for example, the base portion 80 and may be configured not to be removable from the base portion 80.

In this manner, the base portion 80 may include an abutment portion (for example, the wrist abutment portion 92) that limits a lateral movement of the wrist portion including the target region of the subject. For example, the wrist abutment portion 92 may be brought into abutment against the wrist portion including the target region of the subject to limit a lateral movement of the wrist portion (movement in the Z-axis direction illustrated in the drawing).

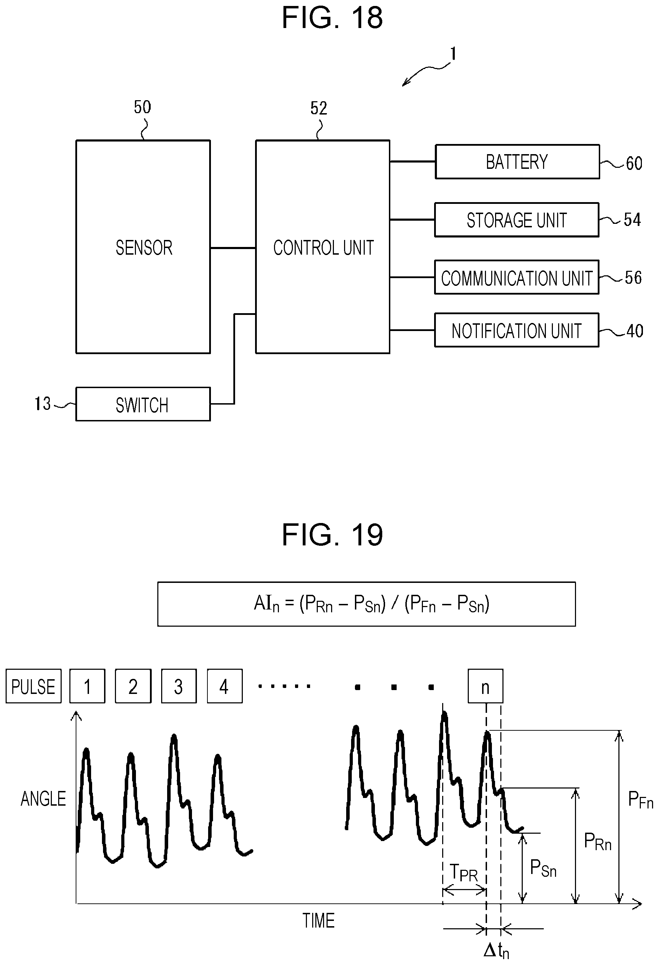

Fig. 18 is a functional block diagram illustrating a schematic configuration of the electronic device 1. The electronic device 1 illustrated in Fig. 18 includes the notification unit 40, the switch 13, the sensor 50, the control unit 52, the storage unit 54, the communication unit 56, and the battery 60. These functional units have been described above.

Fig. 19 is a diagram illustrating an example of a pulse wave acquired at the wrist using the electronic device 1. Fig. 19 illustrates a case where an angular speed sensor is used as the sensor 50 that senses pulsation. Fig. 19 illustrates that an angular speed acquired by the angular speed sensor is integrated with respect to time, in which the horizontal axis represents time, and the vertical axis represents angle. The acquired pulse wave may contain noise caused by, for example, body movement of the subject and may thus be corrected by a filter that removes DC (Direct Current) components to extract only the pulsation components.

A method for calculating the index based on the pulse wave from the acquired pulse wave will be described with reference to Fig. 19. The propagation of the pulse wave is a phenomenon in which a heartbeat caused by blood pumped out of the heart is transmitted through the wall of an artery or the blood. The heartbeat caused by blood pumped out of the heart reaches the periphery of limbs as a forward traveling wave, and a portion thereof is reflected by a blood vessel branch portion, a blood-vessel-diameter changing portion, or the like and returns as a reflected wave. The index based on the pulse wave is, for example, the pulse wave velocity PWV of the forward traveling wave, the magnitude PR of the reflected wave of the pulse wave, the time difference Δt between the forward traveling wave and reflected wave of the pulse wave, the AI (Augmentation Index), which is represented by the ratio of the magnitudes of the forward traveling wave and reflected wave of the pulse wave, or the like.

The pulse wave illustrated in Fig. 19 is a pulse wave with n pulses of a user, where n is an integer greater than or equal to 1. The pulse wave is a composite wave in which a forward traveling wave generated by the ejection of blood from the heart and a reflected wave generated from the blood vessel branch or the blood-vessel-diameter changing portion overlap each other. In Fig. 19, the magnitude of the peak of the pulse wave resulting from the forward traveling wave for each pulse is denoted by P_{Fn}, the magnitude of the peak of the pulse wave resulting from the reflected wave for each pulse is denoted by P_{Rn}, and the minimum value of the pulse wave of each pulse is denoted by P_{Sn}. In Fig. 19, the interval between the peaks of pulses is denoted by T_{PR}.

The index based on the pulse wave is obtained by quantifying information obtained from the pulse wave. For example, the PWV, which is one index based on the pulse wave, is calculated based on the difference in propagation time between pulse waves measured at two target regions such as an upper arm and an ankle and the distance between the two target regions. Specifically, the PWV is calculated by acquiring pulse waves at two points along an artery (for example, an upper arm and an ankle) in synchronization with each other and dividing a distance difference (L) between the two points by a time difference (PTT) between the pulse waves at the two points. For example, as the magnitude PR of the reflected wave, which is one index based on the pulse wave, the magnitude PRn of a peak of the pulse wave resulting from the reflected wave may be calculated, or PRₐᵥₑ obtained by averaging the n magnitudes may be calculated. For example, as the time difference Δt between the forward traveling wave and reflected wave of the pulse wave, which is one index based on the pulse wave, a time difference Δtₙ in a predetermined pulse may be calculated, or Δtₐᵥₑ obtained by averaging the n time differences may be calculated. For example, the AI, which is one index based on the pulse wave, is obtained by dividing the magnitude of the reflected wave by the magnitude of the forward traveling wave, and is expressed by AIₙ = (P_{Rn} - P_{Sn})/(P_{Fn} - P_{Sn}). AIₙ is the AI for each pulse. The AI may be obtained by, for example, measuring a pulse wave for several seconds, calculating an average value AIₐᵥₑ of AIₙ (n is an integer of 1 to n) for the respective pulses, and setting the average value AIₐᵥₑ as an index based on the pulse wave.

The pulse wave velocity PWV, the magnitude P_{R} of the reflected wave, the time difference Δt between the forward traveling wave and the reflected wave, and the AI change depending on the stiffness of the blood vessel wall, and can thus be used to estimate the state of arteriosclerosis. For example, if the blood vessel wall is stiff, the pulse wave velocity PWV is large. For example, if the blood vessel wall is stiff, the magnitude PR of the reflected wave is large. For example, if the blood vessel wall is stiff, the time difference Δt between the forward traveling wave and the reflected wave is small. For example, if the blood vessel wall is stiff, the AI is large. The electronic device 1 can, in addition to estimating the state of arteriosclerosis, estimate blood fluidity (viscosity) using these indices based on the pulse wave. In particular, the electronic device 1 can estimate a change in blood fluidity from a change in the index based on the pulse wave acquired from the same target region of the same subject in a period during which the state of arteriosclerosis does not substantially change (for example, within several days). The blood fluidity represents a measure of the ease of blood flow. For example, if the blood fluidity is low, the pulse wave velocity PWV is small. For example, if the blood fluidity is low, the magnitude PR of the reflected wave is small. For example, if the blood fluidity is low, the time difference Δt between the forward traveling wave and the reflected wave is large. For example, if the blood fluidity is low, the AI is small.

While this embodiment presents an example in which the electronic device 1 calculates the pulse wave velocity PWV, the magnitude PR of the reflected wave, the time difference Δt between the forward traveling wave and the reflected wave, and the AI as example indices based on the pulse wave, the indices based on the pulse wave are not limited thereto. For example, the electronic device 1 may use the posterior systolic blood pressure as an index based on the pulse wave.

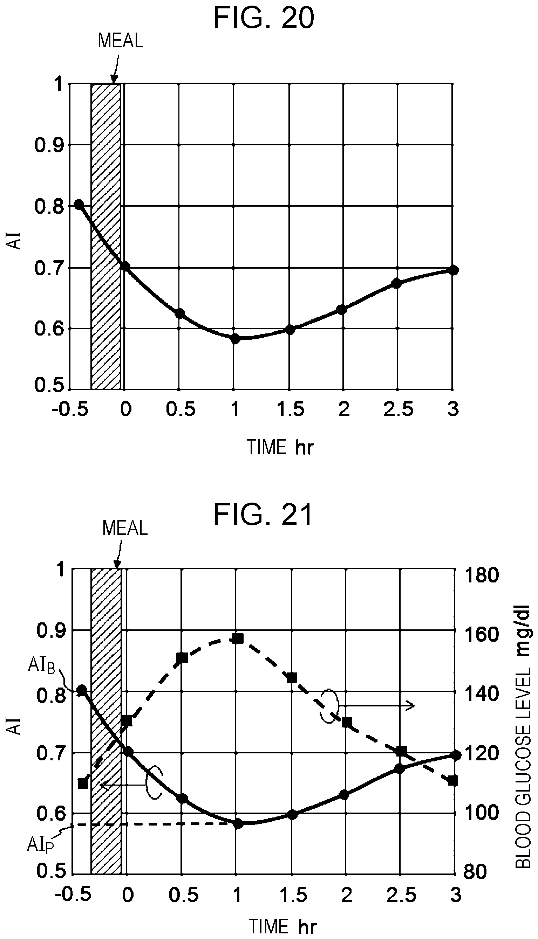

Fig. 20 is a diagram illustrating a time variation in calculated AI. In this embodiment, the pulse wave was acquired for about five seconds using the electronic device 1 including an angular speed sensor 131. The control unit 52 calculated the AI for each pulse from the acquired pulse wave and further calculated the average value AIₐᵥₑ of these AIs. In this embodiment, the electronic device 1 acquired pulse waves at a plurality of timings before and after a meal, and calculated an average value of the AIs (hereinafter referred to as the AI) as an example index based on the acquired pulse waves. In Fig. 20, the horizontal axis represents the passage of time, with the first measurement time after the meal being 0. In Fig. 20, the vertical axis represents the AI calculated from the pulse wave acquired at that time. The pulse waves were acquired over the radial artery while the subject remained at rest.

The electronic device 1 acquired pulse waves every 30 minutes before the meal, immediately after the meal, and after the meal, and calculated a plurality of AIs on the basis of the respective pulse waves. The AI calculated from the pulse wave acquired before the meal was about 0.8. The AI immediately after the meal became smaller than that before the meal, and the AI reached the minimum extreme value at about 1 hour after the meal. The AI gradually increased until the measurement was finished at 3 hours after the meal.

The electronic device 1 can estimate a change in blood fluidity from the change in calculated AI. For example, if red blood cells, white blood cells, and platelets in blood are aggregated together or adhesion increases, blood fluidity decreases. For example, if the water content of plasma in blood becomes low, blood fluidity decreases. These changes in blood fluidity are caused by, for example, the glycolipid state described below or the health condition of the subject, such as heatstroke, dehydration, or hypothermia. Before the health condition of the subject becomes serious, the subject can recognize a change in their blood fluidity by using the electronic device 1 of this embodiment. From the change in AI before and after the meal illustrated in Fig. 20, it can be estimated that the blood fluidity decreased after the meal, the blood fluidity decreased to the lowest level at about 1 hour after the meal, and then the blood fluidity gradually increased. The electronic device 1 may notify the subject of blood fluidity by expressing "thick" for a low blood fluidity state and "thin" for a high blood fluidity state. For example, the electronic device 1 may determine whether the blood is "thick" or "thin" on the basis of the average value of AIs for the age of the subject. The electronic device 1 may determine that the blood is "thin" if the calculated AI is larger than the average value, and determine that the blood is "thick" if the calculated AI is smaller than the average value. The electronic device 1 may determine whether the blood is "thick" or "thin" on the basis of, for example, the AI before the meal. The electronic device 1 may compare the AI after the meal with the AI before the meal and estimate the degree to which the blood is "thick". The electronic device 1 can use, for example, the AI before the meal, that is, the AI on an empty stomach, as an index for the vascular age (vascular stiffness) of the subject. For example, the electronic device 1 calculates an amount of change in calculated AI on the basis of the AI of the subject before the meal, that is, the AI on an empty stomach, thereby making it possible to reduce an estimation error caused by the vascular age (vascular stiffness) of the subject. It is therefore possible to more accurately estimate a change in blood fluidity.

Fig. 21 is a diagram illustrating a calculated AI and a measurement result of blood glucose level. The pulse wave acquisition method and the AI calculation method are the same as those in the embodiment illustrated in Fig. 20. In Fig. 21, the right vertical axis represents blood glucose level in blood, and the left vertical axis represents calculated AI. In Fig. 21, the solid line indicates an AI calculated from an acquired pulse wave, and the dotted line indicates a measured blood glucose level. The blood glucose level was measured immediately after the acquisition of the pulse wave. The blood glucose level was measured using the blood glucose meter "Medisafe Fit", manufactured by Terumo Corporation. Compared to the blood glucose level before the meal, the blood glucose level immediately after the meal increased by about 20 mg/dl. The blood glucose level reached the maximum extreme value at about 1 hour after the meal. Thereafter, the blood glucose level gradually decreased until the measurement was finished, and became almost the same as the blood glucose level before the meal at about 3 hours after the meal.

As illustrated in Fig. 21, the blood glucose level before and after a meal has a negative correlation with the AI calculated from the pulse wave. As the blood glucose level increases, glucose in blood causes aggregation of red blood cells and platelets or increases adhesion, and, as a result, blood fluidity may decrease. A decrease in blood fluidity may decrease the pulse wave velocity PWV. A decrease in pulse wave velocity PWV may increase the time difference Δt between the forward traveling wave and the reflected wave. An increase in the time difference Δt between the forward traveling wave and the reflected wave may cause the magnitude P_{R} of the reflected wave to decrease compared to the magnitude P_{F} of the forward traveling wave. A decrease in the magnitude P_{R} of the reflected wave compared to the magnitude P_{F} of the forward traveling wave may decrease the AI. Since the AI within several hours (in this embodiment, 3 hours) after the meal has a correlation with the blood glucose level, the variation in the blood glucose level of the subject can be estimated from the variation in AI. If the blood glucose level of the subject is measured in advance and the correlation with the AI is acquired, the electronic device 1 can estimate the blood glucose level of the subject from the calculated AI.

The electronic device 1 can estimate the state of glucose metabolism of the subject on the basis of the time of occurrence of the minimum extreme value of the AI detected for the first time after the meal, namely, AI_{P}. The electronic device 1 estimates, for example, the blood glucose level as the state of glucose metabolism. In an example estimation of the state of glucose metabolism, for example, if the minimum extreme value AI_{P} of the AI detected for the first time after the meal is detected after a lapse of a predetermined time or longer (for example, about 1.5 hours or longer after the meal), the electronic device 1 can estimate that the subject has a glucose metabolism disorder (patient with diabetes).

The electronic device 1 can estimate the state of glucose metabolism of the subject on the basis of the difference (AI_{B} - AI_{P}) between AI_{B}, which is the AI before the meal, and AI_{P}, which is the minimum extreme value of the AI detected for the first time after the meal. In an example estimation of the state of glucose metabolism, for example, if (AI_{B} - AI_{P}) is greater than or equal to a predetermined value (for example, greater than or equal to 0.5), it can be estimated that the subject has a glucose metabolism disorder (patient with postprandial hyperglycemia).

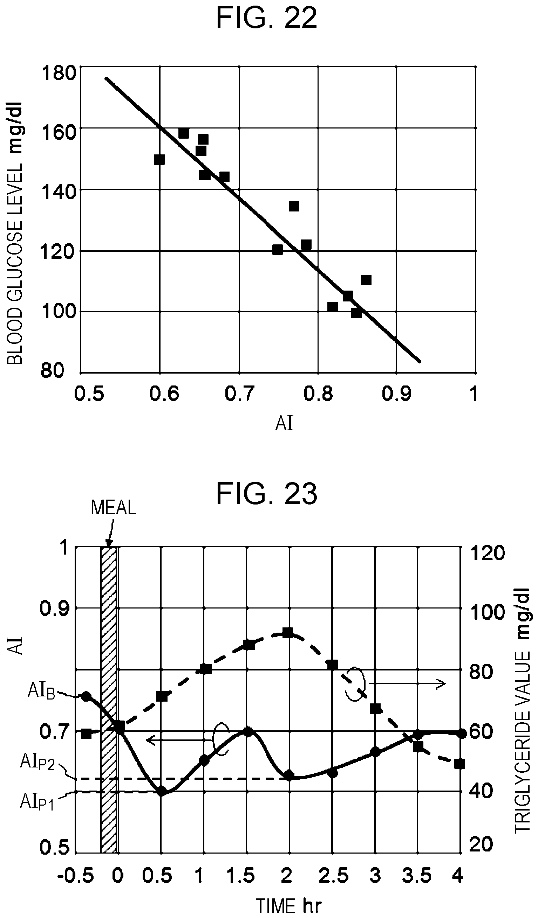

Fig. 22 is a diagram illustrating the relationship between the calculated AI and the blood glucose level. The calculated AI and the blood glucose level were acquired within 1 hour after the meal, within which the blood glucose level varies greatly. The data in Fig. 22 includes a plurality of different pieces of data after the meal for the same subject. As illustrated in Fig. 22, the calculated AI and the blood glucose level exhibited a negative correlation. The correlation coefficient between the calculated AI and the blood glucose level was greater than or equal to 0.9 and exhibited a very high correlation. For example, the correlation between the calculated AI and the blood glucose level illustrated in Fig. 22 may be acquired for each subject in advance, thus allowing the electronic device 1 to estimate the blood glucose level of the subject from the calculated AI.

Fig. 23 is a diagram illustrating a calculated AI and a measurement result of triglyceride value. The pulse wave acquisition method and the AI calculation method are the same as those in the embodiment illustrated in Fig. 20. In Fig. 23, the right vertical axis represents triglyceride value in blood, and the left vertical axis represents AI. In Fig. 23, the solid line indicates an AI calculated from an acquired pulse wave, and the dotted line indicates a measured triglyceride value. The triglyceride value was measured immediately after the acquisition of the pulse wave. The triglyceride value was measured using the lipid measurement device "Pocket Lipid", manufactured by Techno Medica Co., Ltd. Compared to the triglyceride value before the meal, the maximum extreme value of the triglyceride value after the meal increased by about 30 mg/dl. The triglyceride reached the maximum extreme value at about 2 hours after the meal. Thereafter, the triglyceride value gradually decreased until the measurement was finished, and became almost the same as the triglyceride value before the meal at about 3.5 hours after the meal.

In contrast, regarding minimum extreme values of the calculated AI, a first minimum extreme value AI_{P1} was detected at about 30 minutes after the meal, and a second minimum extreme value AI_{P2} was detected at about 2 hours after the meal. The first minimum extreme value AI_{P1} detected at about 30 minutes after the meal can be estimated to be caused by the influence of the blood glucose level after the meal described above. The time of occurrence of the second minimum extreme value AI_{P2} detected at about 2 hours after the meal substantially matches the time of occurrence of the maximum extreme value of the triglyceride detected at about 2 hours after the meal. From this, it can be estimated that the second minimum extreme value AI_{P2} detected after a predetermined time or longer from the meal is caused by the influence of triglyceride. Like the blood glucose level, it was found that the triglyceride value before and after a meal had a negative correlation with the AI calculated from the pulse wave. In particular, since the minimum extreme value AI_{P2} of the AI, which is detected after a predetermined time or longer (in this embodiment, about 1.5 hours or longer) from the meal, has a correlation with the triglyceride value, the variation in the triglyceride value of the subject can be estimated from the variation in AI. If the triglyceride value of the subject is measured in advance and the correlation with the AI is acquired, the electronic device 1 can estimate the triglyceride value of the subject from the calculated AI.

The electronic device 1 can estimate the state of lipid metabolism of the subject on the basis of the time of occurrence of the second minimum extreme value AI_{P2} detected after the predetermined time or longer after the meal. The electronic device 1 estimates, for example, a lipid value as the state of lipid metabolism. In an example estimation of the state of lipid metabolism, for example, if the second minimum extreme value AI_{P2} is detected after a lapse of a predetermined time or longer (for example, 4 hours or longer) after the meal, the electronic device 1 can estimate that the subject has a lipid metabolism disorder (patient with hyperlipidemia).

The electronic device 1 can estimate the state of lipid metabolism of the subject on the basis of the difference (AI_{B} - AI_{P2}) between AI_{B}, which is the AI before the meal, and the second minimum extreme value AI_{P2} detected after the predetermined time or longer after the meal. In an example estimation of lipid metabolism disorder, for example, if (AI_{B} - AI_{P2}) is greater than or equal to 0.5, the electronic device 1 can estimate that the subject has a lipid metabolism disorder (patient with postprandial hyperlipidemia).

From the measurement results illustrated in Fig. 21 to Fig. 23, the electronic device 1 of this embodiment can estimate the state of glucose metabolism of the subject on the basis of the first minimum extreme value AI_{P1} detected earliest after the meal and the time of occurrence of the first minimum extreme value AI_{P1}. The electronic device 1 of this embodiment can further estimate the state of lipid metabolism of the subject on the basis of the second minimum extreme value AI_{P2} detected after a predetermined time or longer after the detection of the first minimum extreme value AI_{P1} and the time of occurrence of the second minimum extreme value AI_{P2}.

In this embodiment, triglyceride has been described as an example estimation of lipid metabolism, the estimation of lipid metabolism is not limited to triglyceride. The lipid value estimated by the electronic device 1 includes, for example, total cholesterol, good (HDL: High Density Lipoprotein) cholesterol, bad (LDL: Low Density Lipoprotein) cholesterol, and the like. These lipid values also exhibit tendencies similar to that for triglyceride described above.

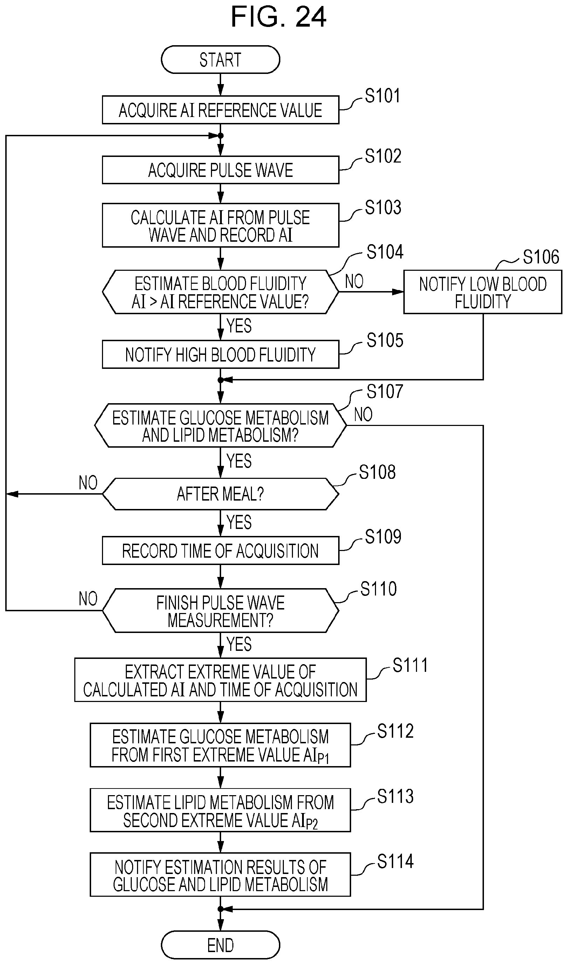

Fig. 24 is a flowchart illustrating a procedure for estimating blood fluidity and the states of glucose metabolism and lipid metabolism on the basis of the AI. Referring to Fig. 24, the flow of estimation of blood fluidity and the states of glucose metabolism and lipid metabolism using the electronic device 1 according to the embodiment will be described.

As illustrated in Fig. 24, the electronic device 1 acquires an AI reference value of the subject as an initial setting (step S101). The AI reference value may be implemented using an average AI estimated from the age of the subject or the AI of the subject on an empty stomach that is acquired in advance. The electronic device 1 may set the AI determined to be before the meal in steps S102 to S108 as the AI reference value, or may set the AI calculated immediately before pulse wave measurement as the AI reference value. In this case, the electronic device 1 executes step S101 after steps S102 to S108.

Then, the electronic device 1 acquires a pulse wave (step S102). For example, the electronic device 1 determines whether a predetermined amplitude or more is obtained for a pulse wave acquired for a predetermined measurement time (for example, 5 seconds). If the predetermined amplitude or more is obtained for the acquired pulse wave, the process proceeds to step S103. If the predetermined amplitude or more is not obtained, step S102 is repeatedly performed (these steps are not illustrated). For example, upon detecting a pulse wave having the predetermined amplitude or more in step S102, the electronic device 1 automatically acquires the pulse wave.

The electronic device 1 calculates, from the pulse wave acquired in step S102, an AI as an index based on the pulse wave and stores the AI in the storage unit 54 (step S103). The electronic device 1 may calculate an average value AIₐᵥₑ from the AIₙ (n is an integer of 1 to n) every predetermined pulse rate (for example, three pulses), and set the average value AIₐᵥₑ as the AI. Alternatively, the electronic device 1 may calculate the AI at a specific pulse.

The AI may be calculated by, for example, performing correction based on a pulse rate P_{R}, a pulse pressure (P_{F} - P_{S}), a body temperature, the temperature of the detected portion, and so on. It is known that there is a negative correlation between the pulse and the AI and between the pulse pressure and the AI and that there is a positive correlation between the temperature and the AI. In order to perform correction, for example, in step S103, the electronic device 1 calculates the pulse and the pulse pressure in addition to the AI. For example, the sensor 50 may include a temperature sensor, and the electronic device 1 may acquire the temperature of the detected portion when acquiring the pulse wave in step S102. The AI is corrected by substituting the acquired pulse, pulse pressure, temperature, and so on into a correction formula created in advance.

Then, the electronic device 1 compares the AI reference value acquired in step S101 with the AI calculated in step S103 and estimates the blood fluidity of the subject (step S104). If the calculated AI is larger than the AI reference value (in the case of YES), it is estimated that the blood fluidity is high, and the electronic device 1 notifies the subject that, for example, the blood fluidity is high (step S105). If the calculated AI is not larger than the AI reference value (in the case of NO), it is estimated that the blood fluidity is low, and the electronic device 1 notifies the subject that, for example, the blood fluidity is low (step S106).

Then, the electronic device 1 asks the subject whether to estimate the states of glucose metabolism and lipid metabolism (step S107). If none of glucose metabolism and lipid metabolism is to be estimated in step S107 (in the case of NO), the electronic device 1 ends the process. If glucose metabolism and lipid metabolism are to be estimated in step S107 (in the case of YES), the electronic device 1 asks the subject whether the calculated AI is acquired before or after the meal (step S108). If the calculated AI is not acquired after the meal (the calculated AI is acquired before the meal) (in the case of NO), the process returns to step S102, and the next pulse wave is acquired. If the calculated AI is acquired after the meal (in the case of YES), the electronic device 1 stores the time at which the pulse wave corresponding to the calculated AI is acquired (step S109). If the pulse wave is to be continuously acquired (in the case of NO in step S110), the process returns to step S102, and the next pulse wave is acquired. If the pulse wave measurement is to be finished (in the case of YES in step S110), the process proceeds to step S111 and the subsequent steps, and the electronic device 1 estimates the states of glucose metabolism and lipid metabolism of the subject.

Then, the electronic device 1 extracts a minimum extreme value and the time thereof from a plurality of AIs calculated in step S104 (step S111) For example, if the AI indicated by the solid line in Fig. 23 is calculated, the electronic device 1 extracts the first minimum extreme value AI_{P1} at about 30 minutes after the meal and the second minimum extreme value AI_{P2} at about 2 hours after the meal.

Then, the electronic device 1 estimates the state of glucose metabolism of the subject from the first minimum extreme value AI_{P1} and the time thereof (step S112). The electronic device 1 further estimates the state of lipid metabolism of the subject from the second minimum extreme value AI_{P2} and the time thereof (step S113). An example estimation of the states of glucose metabolism and lipid metabolism of the subject is similar to that in Fig. 23 described above and will not thus described.

Then, the electronic device 1 notifies the subject of the estimation results obtained in step S112 and step S113 (step S114), and then ends the process illustrated in Fig. 24. The notification unit 40 provides a notification such as "glucose metabolism is normal", "glucose metabolism abnormality is suspected", "lipid metabolism is normal", or "lipid metabolism abnormality is suspected". In this case, the notification unit 40 may provide the notification described above by, for example, turning on or blinking the light-emitting unit. Alternatively, the notification unit 40 may notify the subject of advice such as "You are advised to visit the hospital" or "You are advised to improve your diet". Then, the electronic device 1 ends the process illustrated in Fig. 24.

In this embodiment, the electronic device 1 can estimate the blood fluidity and the states of glucose metabolism and lipid metabolism of the subject from the indices based on the pulse wave. Accordingly, the electronic device 1 can estimate the blood fluidity and the states of glucose metabolism and lipid metabolism of the subject in a noninvasive manner and in a short time.

In this embodiment, the electronic device 1 can perform the estimation of the state of glucose metabolism and the estimation of the state of lipid metabolism from extreme values of an index based on the pulse wave and the times thereof. Accordingly, the electronic device 1 can estimate the states of glucose metabolism and lipid metabolism of the subject in a noninvasive manner and in a short time.

In this embodiment, the electronic device 1 can estimate the states of glucose metabolism and lipid metabolism of the subject on the basis of, for example, the index based on the pulse wave before the meal (on an empty stomach). Accordingly, it is possible to accurately estimate the blood fluidity and the states of glucose metabolism and lipid metabolism of the subject without taking into consideration the blood vessel diameter, the vascular stiffness, or the like, which does not change in a short time.

In this embodiment, the electronic device 1 performs calibration between the index based on the pulse wave and the blood glucose level and between the index based on the pulse wave and the lipid value, thereby being capable of estimating the blood glucose level and the lipid value of the subject in a noninvasive manner and in a short time.

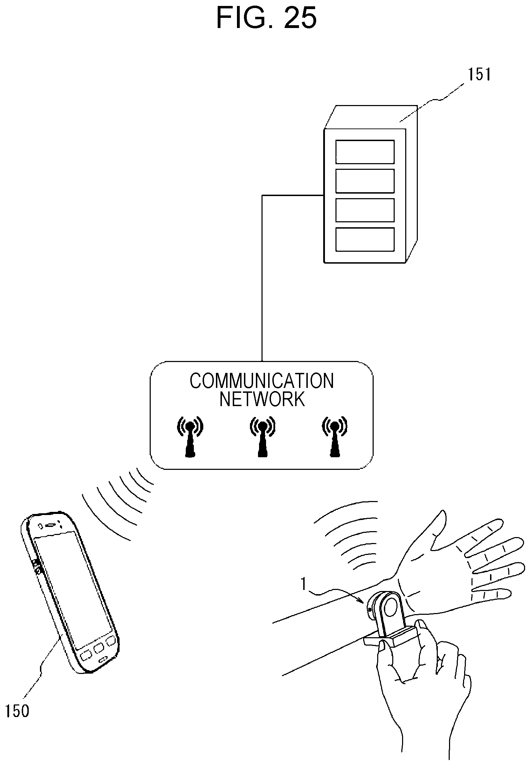

Fig. 25 is a schematic diagram illustrating a schematic configuration of a system according to an embodiment. The system illustrated in Fig. 25 is configured to include the electronic device 1, a server 151, a mobile terminal 150, and a communication network. As illustrated in Fig. 25, an index based on the pulse wave calculated by the electronic device 1 is transmitted to the server 151 via the communication network and is saved in the server 151 as personal information of the subject. The server 151 compares the index based on the pulse wave with previously acquired information of the subject and/or various databases to estimate the blood fluidity and the states of glucose metabolism and lipid metabolism of the subject. The server 151 further creates optimum advice for the subject. The server 151 returns the estimation results and the advice to the mobile terminal 150 possessed by the subject. The mobile terminal 150 notifies the subject of the received estimation results and advice through a display unit of the mobile terminal 150. Such a system can be constructed. Using the communication function of the electronic device 1 enables the server 151 to collect information from a plurality of users, resulting in a further increase in the accuracy of estimation. Since the mobile terminal 150 is used as a notification means, the electronic device 1 no longer requires the notification unit 40 and is further reduced in size. Further, since the server 151 estimates the blood fluidity and the states of glucose metabolism and lipid metabolism of the subject, the computational load on the control unit 52 of the electronic device 1 can be reduced. Further, since previously acquired information of the subject can be saved in the server 151, the load on the storage unit 54 of the electronic device 1 can be reduced. This results in further reduction in the size and complexity of the electronic device 1. In addition, the computational processing speed is also improved.

While the configuration of the system according to this embodiment has been described in which the electronic device 1 and the mobile terminal 150 are connected to each other via the server 151 over the communication network, a system according to the present invention is not limited to this. The electronic device 1 and the mobile terminal 150 may be directly connected to each other over the communication network without using the server 151.

Characteristic examples have been described to fully and clearly disclose the present disclosure. However, the appended claims are not to be limited to the embodiment described above, but are to be configured to embody all modifications and alternative configurations that may be created by a person skilled in the art in this technical field within the scope of the basic matter described herein.

For example, in the embodiment described above, a case has been described in which an angular speed sensor is provided as the sensor 50. However, the form of the electronic device 1 is not limited to this. The sensor 50 may include an optical pulse wave sensor including a light-emitting unit and a light-receiving unit, or may include a pressure sensor. In addition, the target region to be subjected to measurement of biological information by the electronic device 1 is not limited to the wrist of the subject. It is sufficient that the sensor 50 be placed over an artery, such as in a neck, an ankle, a thigh, or an ear.

For example, in the embodiment described above, the states of glucose metabolism and lipid metabolism of the subject are estimated on the basis of the first extreme value and the second extreme value of the index based on the pulse wave and the times thereof. However, the processing executed by the electronic device 1 is not limited to this. In some cases, only either extreme value may appear, or no extreme value may appear. The electronic device 1 may estimate the states of glucose metabolism and lipid metabolism of the subject on the basis of the overall tendency (for example, an integral value, Fourier transform, etc.) of the time variation in the index based on the calculated pulse wave. The electronic device 1 may estimate the states of glucose metabolism and lipid metabolism of the subject on the basis of a time range in which the index based on the pulse wave is less than or equal to a predetermined value, instead of by extracting extreme values of the index based on the pulse wave.

For example, in the embodiment described above, a case has been described in which the blood fluidity before and after a meal is estimated. However, the processing executed by the electronic device 1 is not limited to this. The electronic device 1 may estimate the blood fluidity before and after exercise and during exercise, or may estimate the blood fluidity before and after bathing and during bathing.

In the embodiment described above, the electronic device 1 measures the pulse wave. However, the pulse wave may not necessarily be measured by the electronic device 1. For example, the electronic device 1 may be connected to an information processing device such as a computer or a mobile phone in a wired or wireless manner, and angular speed information acquired by the sensor 50 may be transmitted to the information processing device. In this case, the information processing device may measure the pulse wave on the basis of the angular speed information. The information processing device may execute processing for estimating glucose metabolism and lipid metabolism, or the like. In a case where the information processing device connected to the electronic device 1 executes various types of information processing, the electronic device 1 may not include the control unit 52, the storage unit 54, the notification unit 40, or the like. In a case where the electronic device 1 is connected to the information processing device in a wired manner, the electronic device 1 may not include the battery 60 and may be supplied with power from the information processing device.

In one embodiment, the shapes of the housing 10, the support portion 20, the base portion 80, and so on of the electronic device 1 are not limited to those illustrated in Fig. 3 to Fig. 6. For example, in one embodiment, the housing 10 of the electronic device 1 may be configured to have a shape such as a disk or a triangle. The support portion 20 and the base portion 80 of the electronic device 1 may have any shape such that the support portion 20 and the base portion 80 can be placed on the horizontal surface 100. In one embodiment, the electronic device 1 may have various configurations including a housing including, at least in part, the sensor 50; the support portion 20 that supports the housing 10 on a side thereof; the elastic member 70 interposed between the housing 10 and the support portion 20; and the base portion 80 that supports the support portion 20.

The control unit 52 of the electronic device 1 may estimate at least any one of glucose and lipid metabolism, blood glucose level, and lipid value from the index of the pulse wave. The electronic device 1 may function as a diet monitor that monitors the progress of a diet of the subject or a blood glucose meter that monitors the blood glucose level of the subject.

### Reference Signs List

- 1: electronic device
- 10: housing
- 11: first abutment portion
- 12: second abutment portion
- 13: switch
- 14: protruding portion
- 20: support portion
- 22: rear surface portion
- 24: extension portion
- 26: receiving portion
- 30: substrate
- 40: notification unit
- 50: sensor
- 52: control unit
- 54: storage unit
- 56: communication unit
- 60: battery
- 70: elastic member
- 80: base portion
- 90: wrist rest portion
- 92: wrist abutment portion
- 150: mobile terminal
- 151: server

## Claims

1. An electronic device comprising:
a sensor capable of detecting pulsation in a target region of a subject;
a housing including, at least in part, the sensor;
a support portion that supports the housing on a side thereof;
an elastic member interposed between the housing and the support portion; and
a base portion that allows the support portion to stand upright.

2. The electronic device according to Claim 1, wherein the base portion allows the support portion to stand upright such that the electronic device is erected on a horizontal surface in a free-standing manner.

3. The electronic device according to Claim 1 or 2, wherein the base portion includes a wrist rest portion on which a wrist portion including the target region of the subject is placeable.

4. The electronic device according to Claim 3, wherein a portion of the wrist rest portion where the wrist portion is to be placed is configured to have elasticity.

5. The electronic device according to Claim 4, wherein the portion of the wrist rest portion where the wrist portion is to be placed is formed with slits.

6. The electronic device according to Claim 1 or 2, wherein the base portion includes an abutment portion that limits a lateral movement of a wrist portion including the target region of the subject.

7. The electronic device according to Claim 2, wherein a bottom surface portion of the base portion is formed into a shape such that the electronic device is erected on the horizontal surface in a free-standing manner.

8. The electronic device according to Claim 2 or 7, wherein a center of gravity of the electronic device is positioned such that the electronic device is erected on the horizontal surface in a free-standing manner.

9. The electronic device according to Claim 1, wherein a free-standing erection angle of the support portion relative to a horizontal surface is adjustable.

10. The electronic device according to Claim 1 or 2, wherein a bottom surface portion of the base portion has two or more flat surfaces having different inclination angles.

11. The electronic device according to Claim 10, wherein the bottom surface portion of the base portion is formed into a shape such that the electronic device is erected in a free-standing manner with at least one of the two or more flat surfaces coming into contact with a horizontal surface.

12. The electronic device according to Claim 10 or 11, wherein a center of gravity of the electronic device is positioned such that the electronic device is erected in a free-standing manner with at least one of the two or more flat surfaces coming into contact with a horizontal surface.

13. The electronic device according to any of Claims 1 to 12, further comprising
a stopper that allows the housing to partially abut against the support portion in response to the housing being displaced with respect to the support portion due to a deformation of the elastic member.

14. The electronic device according to Claim 13, wherein the stopper includes a protruding portion formed in one of the housing and the support portion, and a receiving portion formed in the other of the housing and the support portion, and
wherein the receiving portion is configured such that the protruding portion is receivable in the receiving portion.

15. The electronic device according to any of Claims 1 to 14, wherein the support portion is configured to be extendable or contractible stepwise in a predetermined direction.

16. The electronic device according to Claim 15, wherein the support portion is configured to be extendable or contractible in the predetermined direction to make a position of the housing in a height direction adjustable.

17. The electronic device according to Claims 1 to 16, wherein the housing includes a first abutment portion to be brought into abutment against the target region, and a second abutment portion to be brought into abutment against a portion near a location of the target region against which the first abutment portion abuts.

18. The electronic device according to Claim 17, wherein the first abutment portion protrudes from the housing more than the second abutment portion.

19. The electronic device according to any of Claims 1 to 18, wherein the elastic member is deformable in accordance with the pulsation in the target region.

20. The electronic device according to any of Claims 1 to 19, wherein the elastic member is three-dimensionally deformable.

21. The electronic device according to any of Claims 1 to 20, wherein the elastic member is elastically deformed to such an extent that the pulsation in the target region is detectable by the sensor.

22. The electronic device according to any of Claims 1 to 21, wherein the sensor detects the pulsation in the target region as a rotational movement about a predetermined axis.

23. The electronic device according to Claim 22, wherein the sensor detects the pulsation in the target region as rotational movements on at least two axes.

24. The electronic device according to Claim 23, wherein the sensor detects the pulsation in the target region as rotational movements on three axes.

25. The electronic device according to any of Claims 1 to 24, wherein the sensor is a gyro sensor.

26. The electronic device according to any of Claims 1 to 25, further comprising
a control unit that calculates an index of a pulse wave based on the pulsation detected by the sensor, wherein
the control unit combines results detected by the sensor as rotational movements on at least two axes.

27. The electronic device according to Claim 26, wherein the control unit combines only results having components greater than or equal to a predetermined threshold among the results detected by the sensor as rotational movements on at least two axes.

28. The electronic device according to Claim 26 or 27, wherein the control unit combines the results detected by the sensor as rotational movements on at least two axes after polarities of the results are made to match each other.

29. The electronic device according to any of Claims 1 to 28, wherein the elastic member is an elastic member deformable along at least any one axis among three axes orthogonal to one another.

30. The electronic device according to any of Claims 26 to 29, wherein the control unit estimates at least any one of glucose and lipid metabolism, a blood glucose level, and a lipid value from the index of the pulse wave.

31. The electronic device according to any of Claims 1 to 30, wherein the electronic device functions as a diet monitor that monitors a progress of a diet of the subject or a blood glucose meter that monitors a blood glucose level of the subject.
